# EUROPEAN PATENT APPLICATION

(11) **EP 4 763 143 A1**
(43) Date of publication of application: **24.06.2026**
(21) Application number: 25225915.5
(22) Date of filing: 19.12.2025
(51) Int. Cl.: A61F 2/26, A61F 2/00

(54) **A SYSTEM AND AN ELECTRONIC DEVICE FOR TREATMENT OF ERECTILE DYSFUNCTION**

(30) Priority: 20.12.2024 US 202463736817 P; 16.04.2025 US 202563789910 P
(71) Applicant: Coloplast A/S, 3050 Humlebaek (DK)
(72) Inventor: Allen, John J., Mendota Heights (US); Hamlin, Frederick William, Cambridge (GB); Gardner, Steven David, Cambridgeshire, PE2 6FX (GB)

(57) **Abstract**

A body implantable reservoir assembly (114) has a reservoir body (116) adapted to contain the liquid and an electronic assembly (118) disposed in the reservoir body and adapted to move the liquid between the reservoir body and an implantable prosthesis. The electronic assembly has a printed circuit board and a battery hermetically sealed inside a canister, a pump assembly having a motor electrically coupled with the printed circuit board and the battery, a pump coupled with the motor, and a valve assembly coupled with the pump. When the reservoir body contains the liquid, the motor, the pump, and the valve assembly are immersed in the liquid.

## Description

### Background

An implanted penile prosthetic is effective in relieving male erectile dysfunction.

A penile prosthesis (a device is a prosthesis prior to implantation, and a prosthetic after implantation) could be a malleable and non-inflatable device or an inflatable device.

The malleable device has a flexible core that is manually bendable to various orientations, which allows the user to bend the penile implant to a desired erect shape or to a relaxed state.

The inflatable device typically includes a pair of inflatable bladders implantable in the penis, a reservoir implantable in the abdomen, and a pump implantable in the scrotum that is employed to move liquid from the reservoir into the inflatable bladders. Each of the inflatable bladders has a distal portion that is implantable within a dilated corpus cavernosum of the penis and a proximal portion that is implantable within a dilated crus penis. Manually pumping a bulb of the pump moves liquid from the reservoir into the inflatable bladders to gradually fill the inflatable bladders with the liquid. Manually squeezing the pump bulb between about 15-30 times will inflate the inflatable bladders with the liquid sufficiently to create an erection suitable for penetrative intercourse.

Some users have difficulty in manually squeezing the pump bulb enough times to achieve an erection. Some surgeons note that the placement of the pump and its pump bulb in the scrotum presents challenges to users with limited dexterity.

Surgeons and users would benefit from penile prostheses that ease the manual effort in creating an erection to address erectile dysfunction.

### Brief Description of the Drawings

The accompanying drawings are included to provide a further understanding of embodiments and are incorporated in and constitute a part of this specification. The drawings illustrate embodiments and together with the description teach principles of this disclosure. Other embodiments and the intended advantages of embodiments will be readily appreciated as they become better understood by reference to the following detailed description. The elements of the drawings are not necessarily to scale relative to each other. Like reference numerals designate corresponding similar parts.
Fig. 1 is a schematic view of one embodiment of an electronic system useful in the treatment of erectile dysfunction.
Fig. 2 is a schematic view of one embodiment of a physician interface adapted to communicate with an implantable device of the electronic system of Fig. 1.
Fig. 3 is a perspective view of one embodiment of a fob adapted to remotely control or communicate with the implantable device of the electronic system of Fig, 1.
Fig. 4A is a schematic view of one embodiment of an external recharge system adapted to wirelessly transmit energy to the implantable device of the electronic system of Fig. 1.
Fig. 4B is a schematic view of another embodiment of a body-worn external recharge system with a tethered transmit coil adapted to wirelessly transmit energy to the implantable device of the electronic system of Fig. 1.
Fig. 5 is a perspective view of one embodiment of an electronic inflatable penile prosthesis (eIPP).
Fig. 6 is a perspective view of one embodiment of an inflatable penile implant of the eIPP shown in Fig. 5.
Fig. 7 is a perspective view of one embodiment of a reservoir assembly of the eIPP shown in Fig. 5.
Fig. 8 is an exploded perspective view of the reservoir assembly shown in Fig. 7.
Fig. 9 is an exploded perspective view of an electronic assembly of the reservoir assembly shown in Fig. 7.
Fig. 10A is a top view and Fig. 10B is a bottom view of one embodiment of a printed circuit board of the electronic assembly shown in Fig. 9.
Fig. 10C is a schematic view of an architecture of the electronic assembly shown in Fig. 9.
Fig. 11 is a hydraulic symbol diagram representation of the eIPP of Fig. 5.
Fig. 12A is a perspective view of a pump assembly of the eIPP of Fig. 5 and Fig. 12B is another perspective view of a pump assembly of the eIPP of Fig. 5.
Fig. 13A is an exploded perspective view of a pump and a motor of the pump assembly of Figs. 12A-12B, and Fig. 13B is a cross-sectional view of the pump and the motor of FIG. 13A.
Fig. 14A is a cross-sectional view of one alternative embodiment of a pump and a first valve manifold of a pump assembly with the pump off.
Fig. 14B is a cross-sectional view of the alternative embodiment of the pump and the first valve manifold with the pump on and operating to move fluid from the reservoir to the inflatable bladders.
Fig. 14C is a cross-sectional view of the alternative embodiment of the pump and the first valve manifold with the pump on and operating to move fluid from the inflatable bladders to the reservoir.
Fig. 14D is a perspective view of a membrane assembly of the first valve manifold of FIG. 14A.
Fig. 14E is a perspective view of a poppet of the first valve manifold of Fig. 14A.
Fig. 15 is a cross-sectional view of another embodiment of a motor for the pump assembly of Figs. 12A-12B.
Figs. 16A-16F are cross-sectional views of a second valve manifold of the pump assembly of Figs. 12A-12B.
Fig. 16G is a perspective view of a poppet of the second valve manifold of Figs. 16A-F.
Fig. 17 is a perspective view of one embodiment of an electronic artificial sphincter.
Fig. 18A is a side view and Fig. 18B is a top view of a cuff of the electronic artificial sphincter of Fig. 17.
Fig. 19 is a piping and instrumentation diagram representation of the electronic artificial sphincter of Fig. 17.

### Summary

An electronic system and its method of use provides an electronically controllable inflatable prosthesis (an electronic inflatable prosthesis) that is implantable into a user, a physician interface to program and communicate with the electronic inflatable prosthesis, a user interface that allows the user to control the electronic inflatable prosthesis, and a recharge assembly that wirelessly charges a power source within the electronic inflatable prosthesis.

In one embodiment, the inflatable prosthesis is a penile prosthesis having one or two inflatable bladders that are attachable to a reservoir assembly having an electronic assembly and a tethered antenna. The inflatable bladder(s) is/are implantable into a penis and the reservoir assembly and antenna are implantable in the body. In this configuration, the electronically controllable inflatable prosthesis is an electronic inflatable penile prosthesis (eIPP).

In another embodiment, the inflatable prosthesis is an inflatable cuff implantable around a urethra, where the inflatable cuff is attachable to a reservoir assembly having an electronic assembly and a tethered antenna. In this configuration, the electronically controllable inflatable prosthesis is an electronic artificial urinary sphincter (eAUS).

In another embodiment, the inflatable prosthesis is an inflatable cuff implantable around an anus, or around an area to support a deficient anal sphincter. The inflatable cuff is attachable to a reservoir assembly having an electronic assembly and a tethered antenna. In this configuration, the electronically controllable inflatable prosthesis is an electronic artificial sphincter for treating fecal incontinence.

Various embodiments of a method, a system, and an electronic device for treatment of erectile dysfunction are described. The electronic system includes an electronic inflatable penile prosthesis (eIPP) that is implantable into a user, a physician interface to program and communicate with the eIPP, a user interface that allows the user to control the eIPP, and a recharge assembly that wirelessly charges a power source within the eIPP.

The electronic artificial sphincter is suitable for implantation in the body with the cuff surrounding the urethra of the patient, in which case the electronic artificial sphincter is an electronic artificial urinary sphincter (eAUS). The electronic artificial sphincter is suitable for implantation in the body with the cuff surrounding the anal sphincter area of the patient, in which case the electronic artificial sphincter is an electronic artificial fecal incontinence sphincter.

The eIPP includes an inflatable penile prosthesis implantable in the penis, a reservoir assembly including a reservoir body adapted to contain a volume of liquid and an electronic assembly inside of the reservoir body, an antenna assembly, and suitable tubing to fluidically and electrically connect the components. The inflatable penile prosthesis includes a pair of inflatable bladders, each of which is implantable in one of the corpus cavernosum on either side of a central septum in the penis. The pair of inflatable bladders is connected by tubing to the reservoir assembly. The electronic assembly inside of the reservoir body of the reservoir assembly includes a motor to run a pump, a printed circuit board with memory and a processor to control the motor, and a power source to power the motor. One embodiment of the power source is a primary battery, and a second embodiment of the power source is a secondary, rechargeable battery. When a rechargeable battery is employed, an antenna assembly is electrically coupled with the electronic assembly, where the antenna assembly is adapted to wirelessly transfer power from an external source to the secondary battery.

The printed circuit board and the power source are hermetically sealed and separated from the motor / pump. The motor and pump are separated from the electronics, and a louvre or channel is provided to allow liquid in the reservoir body access to the motor / pump. Other approaches have the motor isolated away from the liquid where a sealed shaft extends from the motor area, through a partition, and into the area of the liquid to drive the pump. This conventional style of "dry" motor is sealed relative to the partition / shaft interface and is associated with frictional losses and is susceptible to leaking. In contrast, the system and device described in this application immerses or exposes the motor (and the pump) to the liquid to provide a "wet" motor and has the advantage of providing the pump with improved efficiency by removing the shaft seal, which removes the frictional and other losses from the system. Exposing the motor to the liquid removes the challenge of sealing a dry motor from its liquid environment, which eliminates the possibility of damaging a motor in a dry-motor setup if liquid undesirably leaks into the system.

The patient interface allows the patient to initiate inflation through an inflation mode, or to initiate deflation through a deflation mode, or to program or operate the method, the system, and the electronic device to partially inflate the penile implant for a period of time immediately after implantation to acclimate or condition the implant within the penis and aid in the healing process. The method, system, and the electronic device are operable to partially inflate the penile implant for a partial inflation period of time, deflate the penile implant and maintain the deflated state for a deflated period of time, and again to partially fill the penile implant with an amount of liquid for a subsequent inflation period of time. The periods of time may be the same or different. For example, the method, system, and the electronic device is operable to partially inflate the penile implant for a period of about 48 hours during the first week or weeks after implantation to adjust the penis tissue to the new implant and aid in healing, and subsequently deflate the implant; this initial inflation deflation procedure after implantation can be cycled to hold the partial pressure in the penile implant for about 48 hours, deflate the penile implant for 24 hours, and again to partially fill the penile implant with an amount of liquid for about 48 hours. Another inflation deflation procedure after implantation is cycled to hold the partial pressure in the penile implant for a time in a range from 8 to 96 hours, deflate the penile implant for a time in a range from 1 to 48 hours, and again to partially fill the penile implant with an amount of liquid for a time in a range from 8 to 96 hours.

The doctor interface includes the functionality of the patient interface above and further includes access to the system memory to read or record usage data, monitor or interrogate error data, or control the system to operate the motor and the pump to evacuate air and add liquid to the system during implantation, as examples.

In one embodiment, the pump is coupled to a valve assembly, where the valve assembly is provided as a mirrored valve having two similar valves arranged in a back-to-front, back-to-back, front-to-back, or front-to-front arrangement. The mirrored valve arrangement allows both a passive deflate mode and a TOP-OFF status (or sustained pressure mode). A small orifice formed in an exterior wall of the mirrored valve provides an outlet to allow liquid to slowly, and passively, leak out of the mirrored valve assembly and back to the liquid reservoir. Selection of a size of the orifice, as a function of the liquid pressure and viscosity, allows for a calculated, or selected, flow rate of the liquid out of the penile implant back to the reservoir. Embodiments of the orifice formed in a wall of the pump assembly include an orifice size in a range from about 0.0005 inches to about 0.005 inches, with one preferred size of the orifice being 0.001 inches, or 25 micrometers. The orifice formed in an exterior wall of the mirrored valve intentionally creates a slow leak that will eventually deflate the implant if the system is rendered inoperable, which ensures that the penile implant will move from an erect state back to a flaccid state in the unlikely and undesirable situation where the system stops functioning for any reason. The TOP-OFF status operates to continuously check a pressure inside of the penile implant to top-off any lost liquid and maintain rigidity. One embodiment has combined features including the intentional slow leak feature built into the valve assembly to ensure that the liquid inside of the implant can egress to return the implant to its flaccid state and the TOP-OFF feature to add liquid to the implant to ensure pressure is maintained inside the implant as the system functions as intended.

Additional system efficiency and added advantages can be realized by avoiding running the motor under dry conditions at startup, during implantation for example, by providing the reservoir assembly with a septum providing access to the motor / pump compartment. The septum is configured to be pierced by a syringe, where the syringe adds liquid to the motor / pump compartment to pre-lubricate the motor prior to initial startup. The septum may also be used to aspirate air out of the reservoir assembly with a syringe.

The motor operates in a first direction to operate the pump and move liquid from the reservoir assembly, through tubing, and into the inflatable bladders of the penile implant. The motor is operable in a second, opposite direction to operate the pump to move liquid from the penile implants into the reservoir assembly. The motor is operable, intraoperatively by the surgeon, to initially evacuate air contained in the inflatable bladders and the reservoir, which is useful during implantation to ensure that the system has a full complement of liquid and little or no air or air bubbles that might reduce the efficiency of the pump / motor. During implantation, the motor can be operated to draw liquid from a surgical basin or syringe into the evacuated reservoir assembly and the evacuated inflatable bladders. This ensures that the reservoir and the inflatable bladders are substantially empty of air and filled with liquid in preparation for the eventual implantation of the components into the user's body. Immediately prior to implantation into the patient, most of the liquid is removed from the reservoir and the inflatable bladders to aid the placement of the components into the abdomen and corpora cavernosa, respectively.

After the eIPP is implanted, the inflatable bladders of the implant and the reservoir assembly are at atmospheric pressure (about 14.7 absolute pounds-per-square-inch (psi-a) or zero gauge pounds-per-square-inch (psi-g)). The valve module of the reservoir assembly prevents the flow of liquid in either direction when the pump is not running and until an instruction is given by the patient or the surgeon. During use, the patient provides an instruction (an input) to the controller on the printed circuit board of the electronic assembly to operate the motor, which drives the pump assembly to initiate a pressure increase on a downstream side of the valve assembly. The valve assembly allows the liquid to flow through the valve assembly and into the inflatable bladders of the implant, first increasing the volume of liquid inside of the inflatable bladders of the implant and then subsequently increasing the pressure inside of the inflatable bladders of the implant to a pressure above the atmospheric pressure as selected by the patient. For example, the patient interacts with the user interface to pressurize the penile implant by pushing an inflate button, as one example, which causes the pump and the valve assembly to move liquid from the reservoir body into the penile implant until a desired pressure, as indicated by an erection of the penis, is achieved. The desired pressure selected by the patient becomes the set pressure. The controller on the printed circuit board of the electronic assembly senses the pressure in the inflatable bladders and controls the system to adjust the pressure in the inflatable bladders according to the set pressure, or to maintain the pressure of the inflatable bladders at the set pressure, which is sometimes referred to as the hold pressure state. In this manner, the patient has achieved an erection at a desired set pressure, and the system maintains the set pressure until instructed otherwise. Notably, the controller functions to maintain the set pressure in a range of acceptable pressures associated with the erection. The controller, advantageously, does not respond to a pressure spike that might occur if the penis is squeezed, as one example, or might occur if the patient leans against a countertop and applies pressure to the implanted reservoir assembly. In other words, the controller is only aware of the pressure in the penile implant, which provides an advantage in maintaining a desired set pressure in the penile implant even if the reservoir assembly is squeezed or expanded or otherwise has a pressure change. The system, and specifically the controller, operates to add liquid to the inflatable bladders when a sensed pressure in the inflatable bladders has dropped below the set pressure. The system does not respond to pressure increases that might arise due to pressure applied to the reservoir body or to the penile implants.

A further advantage of the eIPP described in this disclosure is that the valve assembly is provided with a passive failsafe leak in the form of an engineered orifice formed in an exterior wall of the valve assembly. The failsafe leak causes liquid to slowly leak out of the inflatable bladders to the reservoir body, gradually decreasing the pressure in the inflatable bladders. If the system is operating as designed, a pressure sensor communicating with the inflatable bladders alerts the controller that the pressure has dropped below a percentage of the desired set pressure selected by the patient (e.g., below 90% of the set pressure) and the controller starts the motor to run the pump and the valve assembly allows the liquid to flow into the inflatable bladders to top-off the pressure in the penile implant to the desired set pressure. Conversely, if a component of the system is not operating optimally, for example if power has been lost, the mechanical failsafe leak will passively allow liquid to drain from the inflatable bladders until the penile implant is unpressurized and returns to approximately atmospheric pressure (zero psi-g).

The patient controls deflation, for example by interacting with the patient interface (fob) to instruct the system to deflate the inflatable bladders. When the system is instructed to deflate, the motor operates in an opposite direction compared to the inflation direction which causes a local pressure drop on the downstream side of the valve assembly, displacing a portion of the valve, and allowing liquid to flow out of the inflatable bladders back to the reservoir body. The liquid leaves the inflatable bladders until the inflatable bladders are in a flaccid state that the user chooses or until the penile implant no longer contains fluid. The controller is in standby mode; the penile implants are no longer erect, although each of the inflatable bladders could contain a residual amount of liquid. This is sometimes referred to as the dormant or standby state.

One advantageous aspect of embodiments of the system described above is that the surgeon can cause the motor to start and operate the pump to draw liquid into the penile implant during the implantation procedure, for example by using a syringe to pressurize a flow of liquid into the reservoir. The motor is operable to cause a pressure difference across the valve assembly, and the valve assembly responds to the pressure difference by filling the reservoir with fluid drawn from the syringe. A similar pressure difference can be established by the motor operating the pump to auto-expel air from the penile implant. That is to say, establishing a pressure difference in one direction relative to the valve assembly will cause the liquid to flow, and the direction of the flow is directed by the valve assembly. The pressure difference can be established by the surgeon intraoperatively as described above.

As noted above, the motor is immersed in the liquid after implantation. The motor spins to operate the pump, and the pump moves the liquid from the reservoir body into the penile implants to create an erection, and from the penile implants back to the reservoir body to return the implants to the flaccid state. As one example, the motor is provided as a brushless DC (BLDC) outrunner motor with a rotor outside of a stator. The rotor has magnets, and the stator has copper coils wound around a lamination of ferrous material; the result of which is, in the presence of an alternating electric field, the rotor spins or rotates around the stator. In comparison, an inrunner motor has the rotor attached to a shaft, and the rotor rotates within the outer perimeter of the stator. The outrunner motor with permanent magnets has more torque at a lower speed compared to a similarly sized inrunner motor (the distance of the location of the magnets to the center axis for the outrunner motor is larger than the distance of the location of the magnets of the inrunner motor). The outrunner motor described above consequently has a torque advantage because the moment arm of the rotor is increased by the distance of the magnets away from the motor axis. One unexpected result is that no transmission gearing is needed between the motor and the pump, specifically the pump can be designed to run at a speed that is near the most efficient motor speed, so the outrunner motor is synergistic with the pump and obviates a transmission.

In one embodiment, the magnets of the motor are high coercivity permanent magnets, with one example being a neodymium magnet of an alloy of neodymium, iron, and boron having a minimum intrinsic coercivity (H_{cJ}) of 30000 Oersteds (2388 kA/m). Magnetic material is generally considered incompatible with an implantable medical device because an implant is not removable without surgical risk attendant to all surgeries and often cannot be shielded prior to exposure to the fields of a magnetic resonance imaging (MRI) machine. The magnetic material of the medical device would have its magnetic direction re-oriented in the presence of the magnetic field of the MRI machine (the magnetic material would no longer have uniformly alternating pole pairs causing the motor to cease functioning), which would undesirably render the medical device inoperable. The magnets and ferrous material of a medical device would be strongly attracted to the magnetic field of the MRI machine causing undesirable heating or an attraction force applied to the implanted device. For these reasons, medical device makers avoid the use of magnetic material in implantable medical devices. Some proposed electronic penile implants disclose piezoelectric motors that avoid the use of magnetic material, at least in part in an attempt to achieve MRI compatibility after device implantation. Consequently, a medical device, and particularly an implantable device having a motor including magnets, is unlikely to comply with the guidance offered by the Food and Drug Administration in the publication titled Testing and Labeling Medical Devices for Safety in the Magnetic Resonance (MR) Environment, originally published May 20, 2021 and reissued October 10, 2023. However, we have discovered a surprising result that a motor including magnetic and ferrous material can be MRI compatible when implanted in a patient undergoing MRI. Specifically, we have discovered a surprising result that a motor with a volume of ferrous material in a range from about 35 - 105 mm³ of permanent neodymium magnets and 72 - 216 mm³ of ferromagnetic material is MRI compatible, under certain condition evaluated by the U.S. Food and Drug Administration (FDA), to a field of 1.5 Tesla for an MRI environment having a spatial gradient of 20 T/m. For an MRI environment of a 3T field with a spatial gradient of 30 T/m, the induced force is three times greater; therefore, a safe limit for the quantity of ferromagnetic and permanent magnetic material in the implant is approximately one third of the amount allowable in a 1.5T field with a spatial gradient of 20 T/m.

An MRI machine has an annular bore formed by a housing that contains a magnet or magnets extending inside the housing from a front of the bore to the back of the bore. The magnets are selected to provide a strong magnetic field and are sometimes formed by many coils of wires through which electricity is passed to create the magnetic field. Coiled wire or windings can be supercooled and provided as superconducting magnets that produce a magnetic field having a magnetic flux density in a range from 0.5 to 7.0 Tesla (5000 to 70,000 gauss). As a point of reference, the Earth's magnetic field is about 0.5 gauss, so a 1.5 T MRI machine has a magnetic field of about 30,000 times greater than the Earth's magnetic field. For this reason, the patient and each person in the MRI suite is carefully screened to remove all metal objects as a 1.5 T magnetic flux density of the MRI machine could potentially cause metal objects to become projectiles and potentially injure the patient or caregiver. Considering the above, it is a surprising result that the motor we have described has a volume of ferrous material (a metal stator, as an example) and a volume of magnet and yet, when present in an implant, is compatible with the magnetic forces associated with magnetic resonance imaging of the patient. Specifically, it is a surprising result that the disclosed motor having ferromagnetic material corresponds with, or will result in, an induced force on the motor falling within safe limits when in the presence of a magnetic field arising from a 1.5 T MRI with a spatial gradient of 20 T/m. In one embodiment as tested, the surprising result is associated with a motor having 72 mm³ of ferromagnetic material (a metal stator, as an example) and about 35 mm³ of permanent neodymium magnets that corresponds with, or will result in, an induced force on the motor falling within safe limits in the presence of a magnetic field arising from a 1.5 T MRI with a spatial gradient of 20 T/m. Calculations suggest a surprising result is further achievable with a motor having about 216 mm³ of ferromagnetic material (a metal stator, as an example) and about 105 mm³ of permanent neodymium magnets that corresponds with, or will result in, an induced force on the motor falling within safe limits in the presence of a magnetic field arising from a 1.5 T MRI with a spatial gradient of 20 T/m.

An MRI is performed on the patient by passing the patient through the annular bore of the MRI machine and exposing the patient to the magnetic field of the MRI machine. For an MRI machine operating at 1.5 T, the patient is likewise exposed to 1.5 T. The magnetic field of the MRI machine aligns protons of the hydrogen atoms in the patient's body. A radiofrequency is applied to momentarily force the aligned protons to spin out of equilibrium. As the protons return to alignment, they emit signals that can be detected by the MRI machine to assemble detailed images of the body's internal structure.

One approach to achieve MRI compatibility for the eIPP is to place the electronics of the eIPP inside of the reservoir body of the reservoir assembly so that the force acting on the electronics in the reservoir assembly arising from the magnetic field of the MRI machine (the MRI force) is less than the weight of the reservoir body plus the liquid in the reservoir body plus the electronics (the weight of the components being the product of local gravity acting on the mass of the components). The result is that the magnetic MRI field force acting on the metal in the eIPP is much less or even negligible compared to the combined weight of the reservoir assembly.

In several embodiments, a motor with permanent neodymium magnets combined with a ferrous stator is disposed inside an internal cavity of a reservoir body of a reservoir assembly of an electronic inflatable penile prosthesis. The motor is sized by weight and volume such that, when exposed to a 1.5 T magnetic field of a magnetic resonance imaging machine with a maximum magnetic spatial gradient of 20 T/m, the motor responds to the 1.5 T field with an induced force. In an embodiment of the reservoir assembly described below, a combined weight of the reservoir assembly is greater than the induced force in the magnetic field, which is acceptable by the FDA, and thus MRI compatible. In other words, the combined weight of the reservoir assembly being the force due to gravity of the device (reservoir + liquid + motor/pump) is greater than the MRI magnetic field induced force, with the result that the motor in the reservoir assembly, even though it contains magnets and metal, is MRI compatible at 1.5 T.

In one embodiment, a wireless power antenna of the electronic inflatable penile prosthesis is implanted near the belt line and just below the skin (i.e., less than 2 cm below skin level) with the reservoir implanted in the submuscular abdominal region. The wireless power antenna resists rolling / bending and thus has improved electromagnetic coupling with an external charger that allows adding charge to the rechargeable battery of the system. Locating the antenna in this manner places the antenna closer to the external recharging coil for improved electromagnetic coupling for adding charge and separates the charging energy from the reservoir assembly to avoid the reservoir assembly possibly creating electrical interference with the antenna and its charging function.

In one embodiment, a method is described whereby the patient / user is instructed to squeeze the penile implants to force the liquid in the implants through the motor / pump and back to the reservoir body. The movement of the liquid moves the motor in reverse, and instead of consuming charge from the battery to move the rotor, the moving liquid spins the motor to add charge to the battery. This method allows an amount of recharging of the battery during each use cycle. One use cycle includes discharging charge from the battery to move the motor in a first forward direction and operate the pump to pass liquid from the reservoir body to the inflatable implants; and subsequently squeezing / forcing the liquid out of the inflatable implants through the pump and the motor, which rotates the motor in a second reverse direction to add charge to the battery.

### Embodiments

A first embodiment provides a reservoir assembly adapted to supply fluid to an implanted penile prosthesis, the reservoir assembly comprising:
a reservoir body sized to contain a volume of liquid movable from the reservoir body into an inflatable bladder of the penile prosthesis;
an end cap sealed to the reservoir body to form an enclosure within the reservoir body; and
an electronic assembly disposed inside the enclosure within the reservoir body, the electronic assembly comprising:
   a printed circuit board and a battery disposed inside a canister,
   a seal secured to an end of the canister to hermetically seal the printed circuit board and the battery inside the canister,
   a pump head assembly comprising a motor electrically coupled with the printed circuit board and the battery, a pump coupled with the motor, and a valve module coupled with the pump;
wherein, when the enclosure of the reservoir body contains the volume of liquid, the electronic assembly is immersed in the liquid.

An aspect of the first embodiment includes wherein, when the enclosure of the reservoir body contains the volume of liquid, the motor is surrounded by the liquid.

An aspect of the first embodiment includes wherein the motor comprises a magnet and, when the enclosure of the reservoir body contains the volume of liquid, the motor and the magnet are surrounded by the liquid. This aspect provides the unexpected and surprising result that viscous drag on the rotor of the motor that could arise due to the components being immersed in liquid does not stall the motor or significantly degrade motor efficiency.

An aspect of the first embodiment includes wherein the motor comprises a rotor comprising a magnet and a stator comprises a ferrous material, and the rotor and the stator are coated with a material that prevents rust formation on the stator.

An aspect of the above embodiment includes wherein the rotor and the stator are coated to resist corrosion. The rotor and the stator may be coated with the same material, or the rotor could be coated with a different corrosion-resistant material than is coated onto the stator. Suitable corrosion-resistant coatings for one or both of the rotor and the stator include parylene, diamond like carbon, gold plating, or nickel plating.

An aspect of the above embodiment includes wherein one or both of the rotor and the stator are coated with epoxy. This aspect provides the unexpected and surprising result that sufficient motor efficiency is maintained even when the rotor and stator, which are small and low-mass components, are coated in epoxy.

An aspect of the first embodiment includes wherein the motor comprises a shaft coupled with the pump, with the motor adapted to rotate in the liquid obviating use of a shaft seal disposed around the shaft.

An aspect of the first embodiment includes wherein the seal comprises a ceramic seal and the canister comprises titanium housing, and the ceramic seal is fitted within the end of the titanium housing to hermetically seal the printed circuit board and the battery inside of the canister.

An aspect of the first embodiment includes wherein the end cap comprises a first passage fluidically connecting the valve module to the inflatable bladder of the penile prosthesis.

An aspect of the first embodiment includes wherein the battery is a rechargeable battery and the end cap comprises a second passage, with the reservoir assembly further comprising:
a wire disposed in the second passage, with the wire electrically connecting the rechargeable battery with the electronics assembly, which is coupled with an antenna located external to the reservoir body.

An aspect of the first embodiment includes wherein the reservoir assembly comprises a septum, the septum adapted to self-seal after a needle passes through the septum to deliver an initial amount of liquid to lubricate the pump upon start-up.

An aspect of the first embodiment includes the reservoir body comprises a septum, the septum adapted to self-seal after a needle passes through the septum to deliver an initial amount of liquid to lubricate the pump upon start-up.

An aspect of the first embodiment includes wherein the end cap comprises a septum, the septum adapted to self-seal after a needle passes through the septum to deliver an initial amount of liquid to lubricate the pump upon start-up.

An aspect of the first embodiment includes wherein the end cap comprises a gripping tab, the gripping tab comprising opposing walls that project away from an external surface of the end cap to form a grip surface adapted to aid implantation and removal of the reservoir assembly.

A second embodiment provides a reservoir assembly adapted to supply fluid to an implanted penile prosthesis, the reservoir assembly comprising:
a reservoir body defining an internal cavity;
an end cap sealed to the reservoir body to enclose the internal cavity;
a canister disposed within the internal cavity, the canister hermetically sealed to contain a printed circuit board and a battery; and
a pump head assembly disposed within the internal cavity, the pump head assembly comprising a motor electrically coupled with the printed circuit board and the battery, a pump coupled with the motor, and a valve module coupled with the pump;
wherein, when liquid is introduced to the internal cavity in the reservoir body, the canister and the pump head assembly are exposed to the liquid.

An aspect of the second embodiment includes wherein the canister is hermetically sealed by an endwall and motor is electrically coupled with the printed circuit board and the battery by electrical wires passed via hermetic feedthroughs in the endwall, with the electrical wires sealed relative to the opening in the endwall to prevent the liquid entering the canister.

A third embodiment provides a reservoir assembly attachable to an electronic implanted penile prosthesis, the reservoir assembly comprising:
a reservoir body defining an internal cavity;
an end cap sealed to the reservoir body to enclose the internal cavity;
an implant volume of liquid contained inside of the internal cavity of the reservoir body;
a canister disposed within the internal cavity of the reservoir body, the canister hermetically sealed to contain a printed circuit board and a battery;
a motor disposed within the internal cavity of the reservoir body, the motor comprising magnetic and ferromagnetic material, the motor electrically coupled with the printed circuit board and the battery;
wherein, in a presence of a magnetic field of a magnetic resonance imaging machine, the magnetic and ferromagnetic material of the motor creates an induced force, and a combined weight of the reservoir assembly is greater than the induced force created by the magnetic and ferromagnetic material of the motor in the magnetic field.

A fourth embodiment provides a method of implanting a penile prosthesis, the method comprising:
providing an electronic inflatable penile prosthesis (eIPP) comprising an antenna assembly electrically attachable to a battery and a circuit board hermetically sealed inside of a reservoir assembly, a pair of inflatable bladders fluidically attachable to the reservoir assembly, and a motor contained inside of the reservoir assembly;
forming an infrapubic incision above a penis superior to a penile pubic junction;
forming bilateral corporotomies and accessing corpora cavernosa of the penis;
inserting the reservoir assembly into the infrapubic incision and placing the reservoir assembly above a navel of an umbilical region;
inserting a first inflatable bladder of the pair of inflatable bladders into a first corpus cavernosum of the penis and inserting a second inflatable bladder of the pair of inflatable bladders into a second corpus cavernosum of the penis;
inserting an antenna assembly into the infrapubic incision and placing the antenna assembly just below skin level and below the navel of the umbilical region; and
adding a liquid into the reservoir assembly and surrounding the motor with the liquid.

An aspect of the fourth embodiment includes wherein forming the infrapubic incision comprises forming one and only one incision in skin away from the penis.

An aspect of the fourth embodiment includes wherein inserting the reservoir assembly into the infrapubic incision comprises inserting an instrument into an inguinal ring, perforating transversalis fascia, and placing the reservoir assembly in a space posterior the transversalis fascia.

An aspect of the fourth embodiment includes wherein inserting the reservoir assembly into the infrapubic incision comprises inserting an instrument into an inguinal ring to form a tunnel and placing the reservoir assembly in the tunnel and anterior the transversalis fascia.

An aspect of the fourth embodiment includes wherein placing the antenna assembly below the navel of the umbilical region comprises tunneling an instrument through the transversalis fascia and inserting the reservoir assembly posterior the pubic bone and inserting the antenna assembly anterior the pubic bone.

An aspect of the fourth embodiment includes wherein placing the antenna assembly below the navel of the umbilical region comprises tunneling an instrument to form an open space a the transversalis fascia and inserting the antenna assembly into the open space and along a belt line of a user of the eIPP.

An aspect of the fourth embodiment includes wherein the motor comprises a permanent magnet and a combined mass of the reservoir assembly including the motor and the liquid and the battery and the circuit board hermetically sealed inside of the reservoir assembly configures the eIPP to be MRI compatible in a magnetic field having a magnetic flux density in a range from 0.5 T to 1.5 T with a maximum spatial gradient of 20 T/m.

A fifth embodiment provides an electronic penile implant adapted to treat erectile dysfunction, the electronic penile implant comprising:
a penile prosthesis implantable into a penis;
a fluid reservoir adapted to retain liquid transferable into the penile prosthesis to inflate the penile prosthesis;
a motor system retained within the fluid reservoir, the motor system comprising:
   a battery,
   a printed circuit board electrically connected to the battery,
   a motor,
   a pump adapted to be powered by the motor to move the liquid out of the reservoir and into the penile prosthesis,
   a pressure sensor adapted to sense a pressure in the penile prosthesis when inflated with the liquid,
   a valve module adapted to control movement of the liquid relative to the reservoir, the valve module provided with an outlet to pass the liquid between the fluid reservoir and the penile prosthesis, and
   a fluid connection between the pressure sensor and the penile prosthesis;
an end cap coupled to the fluid reservoir and to the motor system retained within the fluid reservoir;
tubing connected between the end cap and the penile prosthesis, the tubing providing a flow path for the liquid between the fluid reservoir and the penile prosthesis; and
an antenna coil coupled to the end cap and in electrical communication with the battery, where the antenna coil allows recharging of the battery.

One aspect of the fifth embodiment further comprises a housing adapted to contain and enclose the motor system, wherein the housing includes an orifice adapted to provide a channel to allow liquid in the fluid reservoir to enter through the housing to the motor system.

One aspect of the fifth embodiment includes wherein the antenna coil is coupled in electrical communication with the battery by an electrical lead.

One aspect of the fifth embodiment includes wherein the antenna coil is coupled in electrical communication with the battery by an electrical lead, and the electrical lead includes an IS-1 connector to allow the electrical lead to be disconnected from the battery.

A sixth embodiment provides an electronic penile implant system adapted to treat erectile dysfunction, the electronic penile implant system comprising:
a body implant comprising:
   a penile prosthesis implantable into a penis,
   a fluid reservoir adapted to retain liquid transferable into the penile prosthesis to inflate the penile prosthesis,
   a motor system retained within the fluid reservoir,
   an end cap coupled to the fluid reservoir and to the motor system retained within the fluid reservoir,
   tubing connected between the end cap and the penile prosthesis, the tubing providing a flow path for the liquid between the fluid reservoir and the penile prosthesis,
   an antenna coil coupled to the end cap and in electrical communication with a rechargeable battery, where the antenna coil allows recharging of the battery;
a patient controller adapted for wireless communication with the body implant;
a recharge system adapted to be placed outside of a body of the patient for charging and recharging of the battery; and
a physician controller adapted to allow a physician to program and control the body implant.

One aspect of the sixth embodiment includes wherein the motor system comprises:
a printed circuit board electrically connected to the battery,
a motor,
a pump adapted to be powered by the motor to move the liquid out of the reservoir and into the penile prosthesis,
a pressure sensor adapted to sense a pressure in the penile prosthesis when inflated with the liquid,
a valve module adapted to control movement of the liquid relative to the reservoir, the valve module provided with an outlet to pass the liquid between the fluid reservoir and the penile prosthesis, and
a fluid connection between the pressure sensor and the valve module.

One aspect of the sixth embodiment includes wherein the antenna coil comprises turns of coiled wire, with the turns of coiled wire in a range from 2 turns to 20 turns of coiled wire.

One aspect of the sixth embodiment includes wherein the antenna coil comprises 5 turns of coiled A WG-12 magnet wire with a pitch of 6 mm.

A seventh embodiment provides a fluid reservoir adapted to retain liquid transferable into a penile prosthesis to inflate the penile prosthesis and treat erectile dysfunction, the fluid reservoir comprising:
a container;
a motor system retained inside of the container, the motor system comprising:
   a battery,
   a printed circuit board electrically connected to the battery,
   a motor,
   a pump adapted to be powered by the motor to move the liquid out of the container and into the penile prosthesis,
   a pressure sensor adapted to sense a pressure in the penile prosthesis when inflated with the liquid,
   a valve module adapted to control movement of the liquid relative to the container, the valve module provided with an outlet to pass the liquid between the container and the penile prosthesis, and
   a fluid connection coupled between the pressure sensor and the valve module; and
an end cap coupled to the container and to the motor system retained within the container;
wherein the end cap comprises a tubing connection for connecting the fluid reservoir to the penile prosthesis and an electrical connection for attaching the motor system to a charging coil.

An eighth embodiment provides an electronic unit adapted to move liquid into a penile prosthesis to inflate the penile prosthesis and treat erectile dysfunction, the electronic unit comprising:
a reservoir comprising an outer wall adapted to expand when the reservoir is filled with a volume of liquid and contract when the volume of liquid is expelled from the reservoir; and
an electronic assembly comprising a housing with a portion of the housing inserted into the reservoir, the electronic assembly comprising:
   an energy source;
   a motor connected to the energy source;
   a gear train connected to the motor;
   a pump block connected to the gear;
   an outlet communicating with the pump block; and
   a drain valve formed in the housing and communicating with the pump block;
wherein energizing the motor moves the gear train in a first direction to allow the pump block to draw the volume of liquid into the outlet and pass the volume of liquid through the drain valve to fill the reservoir.

One aspect of the eighth embodiment includes wherein the gear train is a gearbox.

One aspect of the eighth embodiment includes wherein, when the outlet is connected to a penile implant, the motor is adapted to move the gear train in a second direction to allow the pump block to displace a portion of the volume of liquid from the reservoir, through the drain valve, and out of the outlet to the penile implant.

One aspect of the eighth embodiment includes wherein the energy source, the motor, and the gear train are hermetically sealed inside of the housing.

One aspect of the eighth embodiment includes wherein the pump block comprises an enclosure that is retained inside of the housing, and the enclosure of the pump block encloses a pump head and a dynamic rotary shaft seal, with the pump head coupled to the gear across the dynamic rotary shaft seal, wherein the dynamic rotary shaft seal prevents the volume of liquid from contacting the motor and the energy source.

One aspect of the eighth embodiment includes wherein the pump block comprises an enclosure that is retained inside of the housing, where the enclosure of the pump block comprises a pump head, the enclosure comprising:
a dynamic seal providing a liquid-tight seal between the pump head and the gear train disposed in the housing.

One aspect of the eighth embodiment includes wherein the pump block comprises an enclosure that is retained inside of the housing, the enclosure comprising a liquid-tight seal adapted to prevent the volume of liquid from contacting the motor and the energy source and the drain valve that communicates with the reservoir through the enclosure and the housing.

A ninth embodiment relates to a regenerative pump and battery providing a method of operating an inflatable penile prosthesis, the method comprising:
driving a motor-generator to operate a pump to inflate a bladder of the inflatable penile prosthesis; and
manually squeezing the bladder to force fluid through the pump to drive the motor-generator to charge the battery.

An aspect of the ninth embodiment includes wherein driving the motor-generator to operate the pump to inflate the bladder of the inflatable penile prosthesis comprises:
drawing a fluid from a reservoir of the inflatable penile prosthesis into the pump; and
discharging the fluid from pump into the bladder.

An aspect of the ninth embodiment includes wherein manually squeezing the bladder to force fluid through the pump further comprises returning the fluid from the bladder to the reservoir.

An aspect of the ninth embodiment further includes:
receiving an electronic signal at a subdermal receiver coil electrically coupled to the battery; and
responsive to receiving the electronic signal at the subdermal receiver coil, charging the battery.

A tenth embodiment provides an inflatable penile prosthesis system comprising:
a bladder;
a pump fluidly connected to the bladder;
a battery;
a motor-generator operably connected to the pump and electrically connected to the battery; and
a controller having one or more processors and memory storing instructions that, when executed by the one or more processors, cause the one or more processors to:
   operate in a first mode to drive the motor-generator to operate the pump to inflate the bladder; and
   operate in a second mode to charge the battery in response to fluid being manually forced through the pump and driving the motor-generator.

An aspect of the tenth embodiment further includes a valve assembly fluidly coupled between the pump and the bladder.

An aspect of the tenth embodiment includes wherein the valve assembly is configured to allow fluid flow in only one direction at a time, the valve assembly configured to allow flow in the one direction when the associated pilot pressure is greater than or equal to a threshold pilot pressure.

An aspect of the tenth embodiment includes wherein the controller is further configured to operate in a third mode to drive the motor-generator to operate the pump to deflate the bladder.

An aspect of the tenth embodiment further includes a reservoir fluidly coupled to the pump opposite the bladder.

An aspect of the above embodiment includes wherein the bladder, the pump, and the reservoir define a closed loop fluid system.

An eleventh embodiment relates to User Interfaces for an inflatable penile prosthesis system comprising:
a bladder;
a pump fluidly connected to the bladder;
a battery; and
a motor operably connected to the pump and electrically connected to the battery;
a user interface; and
a controller having one or more processors and memory storing instructions that, when executed by the one or more processors, cause the one or more processors to operate the motor to drive the pump to inflate the bladder responsive to receiving an inflation signal from the user interface.

An aspect of the eleventh embodiment includes wherein manual operation of the bladder drives fluid from the bladder through the pump back to the reservoir.

An aspect of the above embodiment includes wherein manual operation of the bladder drives fluid from the bladder bypassing the pump back to the reservoir.

An aspect of the eleventh embodiment includes wherein the user interface comprises an implantable microphone in wired communication with the controller.

An aspect of the eleventh embodiment includes wherein the user interface comprises an implantable device that is responsive to touch inputs in wired communication with the controller.

An aspect of the above embodiment includes wherein the implantable device is configured to send the inflation signal to the controller in response to sensing a tap by a user.

An aspect of the eleventh embodiment includes wherein the controller further comprises an antenna configured to communicate with the user interface when the controller is implanted in a subject and the user interface is external of the subject.

An aspect of the eleventh embodiment includes wherein the controller is further configured to store instructions that, when executed by the one or more processors, cause the one or more processors to:
operate the motor to drive the pump to deflate the bladder responsive to receiving a deflation signal from the user interface.

An aspect of the eleventh embodiment includes wherein the controller is configured to only operate the motor to drive the pump to inflate the bladder only when the inflation signal is continually received from the user interface.

An aspect of the eleventh embodiment includes wherein when the inflation signal is no longer received from the user interface, the controller is configured to maintain a pressure in the bladder with a pressure range.

An aspect of the eleventh embodiment includes wherein when the inflation signal is no longer received from the user interface, the controller stops operation of the motor, allowing the valve assembly to shut, thereby isolating fluid flow.

An aspect of the eleventh embodiment includes wherein the controller is configured to operate the motor to drive the pump to inflate the bladder to a pressure with a pressure range when the inflation signal is received from the user interface.

A twelfth embodiment relates to a motor Submerged in Saline provided by an inflatable penile prosthesis system comprising:
a reservoir comprising a main reservoir chamber that is configured to receive saline;
a motor positioned in the reservoir and in fluid communication with the main reservoir chamber, wherein the motor comprises a rotor and a stator each configured to be in fluid communication with the main reservoir chamber such that, in operation, the saline in the main reservoir chamber can flow into the motor in contact with the rotor and the stator.

An aspect of the twelfth embodiment further includes a bladder; and
a pump operably connected to the motor and configured to move the saline from the reservoir to the bladder.

An aspect of the twelfth embodiment includes wherein the pump is positioned in the reservoir.

An aspect of the twelfth embodiment includes wherein only the input-output port of the pump protrudes externally from the main reservoir chamber.

An aspect of the twelfth embodiment includes wherein the rotor and the stator each comprise material configured to corrode when in contact with saline and wherein the rotor and the stator each comprise protective coatings configured to separate the material from the saline.

An aspect of the twelfth embodiment includes wherein the motor comprises a shaft without a shaft seal.

A thirteenth embodiment relates to the use of the motor to aid implantation through an inflatable penile prosthesis system comprising:
a reservoir;
a bladder;
a pump fluidly connected to the reservoir and the bladder;
a motor operably connected to the pump; and
a controller having one or more processors and memory storing instructions that, when executed by the one or more processors, cause the one or more processors to:
   operate in a first mode to drive the motor to fill the reservoir from an external source of saline; and
   operate in a second mode to drive the motor to pump the saline from the reservoir to the bladder.

An aspect of the thirteenth embodiment further includes a tube extending between the bladder and the pump, the tube configured to receive the saline from the external source.

An aspect of the thirteenth embodiment includes wherein the reservoir comprises a septum configured to receive a syringe.

An aspect of the thirteenth embodiment includes wherein the controller is configured to operate in the first mode during surgical implantation of the inflatable penile prosthesis system by a medical professional and configured to operate in the second mode during later operation of the inflatable penile prosthesis system by a user of the inflatable penile prosthesis system.

A fourteenth embodiment provides a method of operating an inflatable penile prosthesis system, the method comprising:
operating a motor to fill a reservoir from an external source of saline during a procedure including surgical implantation of the inflatable penile prosthesis system by a medical professional; and
operating the motor to pump the saline from the reservoir to the bladder during later operation of the inflatable penile prosthesis system by a user of the inflatable penile prosthesis system.

A fifteenth embodiment provides an inflatable penile prosthesis system comprising:
a reservoir;
a bladder;
a pump fluidly connected to the reservoir and the bladder;
a motor operably connected to the pump; and
a controller having one or more processors and memory storing instructions that when executed by the one or more processors cause the one or more processors to:
   operate in a first mode to drive the motor evacuate air from the reservoir; and
   operate in a second mode to drive the motor to pump saline from the reservoir to the bladder.

An aspect of the fifteenth embodiment includes wherein the controller is configured to operate in the first mode during surgical implantation of the inflatable penile prosthesis system by a medical professional and configured to operate in the second mode during later operation of the inflatable penile prosthesis system by a user of the inflatable penile prosthesis system.

A sixteenth embodiment provides a method of operating an inflatable penile prosthesis system, the method comprising:
operating a motor to evacuate air from the reservoir during a procedure including surgical implantation of the inflatable penile prosthesis system by a medical professional; and
operating the motor to pump the saline from the reservoir to the bladder during later operation of the inflatable penile prosthesis system by a user of the inflatable penile prosthesis system.

A seventeenth embodiment relates to a pump for an eIPP including an integrated and intentional slow leak and provides an inflatable penile prosthesis system comprising:
a reservoir;
a bladder;
a pump fluidly connected to the reservoir and the bladder;
a bypass pathway configured to connect the reservoir to the bladder.

An aspect of the seventeenth embodiment includes wherein the bypass pathway fluidly connects the reservoir to the bladder such that fluid flows in one direction only from the bladder to the reservoir.

An aspect of the seventeenth embodiment includes wherein the pump comprises a main pathway coupled to a valve assembly that is configured to allow the pump to move fluid between the reservoir and the bladder and wherein the bypass pathway comprises an orifice in a wall of the valve assembly that allows fluid flow around the main pathway even when the main pathway is closed.

An aspect of the seventeenth embodiment includes wherein the bypass pathway is unvalved and is always open.

An aspect of the seventeenth embodiment includes wherein the bypass pathway is sized to allow a fully inflated bladder to slowly deflate when the pump is not operated.

An aspect of the seventeenth embodiment includes wherein the bypass pathway is sized to allow a fully inflated bladder to slowly deflate to a pressure of about 1 pounds-per-square-inch (psi) within about 8 hours when the pump is not operated.

An aspect of the seventeenth embodiment further includes a motor operably connected to the pump;
a battery electrically connected to the motor; and
a controller having one or more processors and memory storing instructions that, when executed by the one or more processors, cause the one or more processors to:
   operate in a first mode to drive the motor to pump fluid from the reservoir to the bladder; and
   operate in a second mode that allows fluid to slowly flow from the bladder through the bypass pathway to the reservoir without operating the motor.

An aspect of the seventeenth embodiment includes wherein the valve assembly comprises a piloted check valve and wherein the bypass pathway comprises an orifice in the valve assembly that allows fluid flow around the piloted check valve even when the piloted check valve is closed.

An eighteenth embodiment relates to a brushless (direct current) DC pump that is magnetic resonance (MR) Compatible provided by an inflatable penile prosthesis system comprising:
a reservoir;
a bladder;
a pump fluidly connected to the reservoir and the bladder;
a brushless DC motor operably connected to the pump, wherein the brushless DC motor is MR compatible.

An aspect of the eighteenth embodiment includes wherein brushless DC motor is positioned in the reservoir.

An aspect of the eighteenth embodiment includes wherein the brushless DC motor is made of at least some magnetic material and is positioned in a portion of the inflatable penile prosthesis such that resulting forces applied by a 1.5 Tesla MR machine are less than a threshold force.

An aspect of the eighteenth embodiment includes wherein the brushless DC motor comprises a high magnetic permeability ferromagnetic material stator, magnets, and a high magnetic permeability ferromagnetic material ring positioned around the magnets.

An aspect of the eighteenth embodiment includes wherein the brushless DC motor comprises a steel stator, magnets, and a steel ring positioned around the magnets.

A nineteenth embodiment relates to a Thrust Bearing Hermetic Seal provided by an inflatable penile prosthesis system comprising:
a reservoir;
a bladder;
a pump fluidly connected to the reservoir and the bladder;
a motor having a shaft with first and second ends;
a housing at least partially containing the motor;
a ceramic seal at least partially sealing the housing and defining a thrust bearing surface positioned proximate the first end of the shaft to form a thrust bearing.

An aspect of the nineteenth embodiment includes wherein the housing comprises titanium and wherein the housing further contains the pump, a battery, a sensor, and a PCB (printed circuit board).

An aspect of the nineteenth embodiment includes wherein the ceramic seal is configured to decrease a force of an inductive loop through the motor generated responsive to a magnetic field.

A twentieth embodiment relates to a Motor and Septum of an inflatable penile prosthesis system comprising:
a reservoir;
a bladder;
a pump fluidly connected to the reservoir and the bladder;
a motor operably connected to the pump and configured to move fluid between the reservoir and the bladder; and
a septum comprising silicone, wherein the septum is positioned with respect to the reservoir such that the septum can be pierced with a needle for injecting saline into the reservoir.

An aspect of the twentieth embodiment includes wherein the septum is fluidly connected to the pump by one of a suction and a discharge conduit.

An aspect of the twentieth embodiment includes wherein the septum is connected to the reservoir to pass through a portion of the reservoir.

An aspect of the twentieth embodiment wherein the septum is configured to reseal responsive to removal of the needle.

An aspect of the twentieth embodiment further includes an expandable wire wrap surrounding the septum.

A twenty first embodiment relates to Motor Features of an inflatable penile prosthesis system comprising:
a reservoir;
a bladder;
a pump fluidly connected to the reservoir and the bladder;
a motor operably connected to the pump, the rotor comprising:
   a rotor comprising a plurality of magnets; and
   a stator positioned inside of the rotor.

An aspect of the twenty first embodiment includes wherein the motor is connected to the pump without a transmission between the motor and the pump.

An aspect of the twenty first embodiment includes wherein the magnets are high coercivity magnets configured to resist demagnetization in a 1.5 Tesla magnetic resonance imaging system.

An aspect of the twenty first embodiment includes wherein the magnets are high coercivity magnets configured to resist demagnetization in a 3 Tesla magnetic resonance imaging system.

An aspect of the twenty first embodiment includes wherein the stator is a high magnetic permeability ferromagnetic material, and wherein the rotor comprises a high magnetic permeability ferromagnetic material ring positioned around the magnets.

An aspect of the twenty first embodiment includes wherein the stator is steel and wherein the rotor comprises a steel ring positioned around the magnets.

An aspect of the twenty first embodiment includes wherein the motor further comprises:
a shaft; and
first and second bearings comprising corundum in bearing contact with first and second portions of the shaft.

An aspect of the above embodiment includes wherein the first and second bearings are substantially annular, wherein the first bearing has a substantially cylindrical inner bearing surface, and wherein the second bearing has a substantially curved inner bearing surface like a torus.

An aspect of the twenty first embodiment includes wherein the second bearing is a circular toroid or an ovular toroid.

A twenty second embodiment relates to a body implantable reservoir assembly adapted to move liquid relative to an implantable prosthesis, the reservoir assembly comprising:
a reservoir body adapted to contain the liquid; and
an electronic assembly disposed in the reservoir body and adapted to move the liquid between the reservoir body and the implantable prosthesis, the electronic assembly comprising:
   a printed circuit board and a battery hermetically sealed inside a canister, and
   a pump assembly comprising a motor electrically coupled with the printed circuit board and the battery, a pump coupled with the motor, and a valve assembly coupled with the pump;
wherein, when the reservoir body contains the liquid, the motor, the pump, and the valve assembly are immersed in the liquid.

An aspect of the twenty second embodiment includes wherein the printed circuit board and the battery are hermetically sealed inside the canister by a seal secured to the canister, and the seal comprises an electrical feedthrough that forms an electrical connection between the motor and the printed circuit board and the battery.

An aspect of the twenty second embodiment includes wherein the implantable prosthesis comprises a penile implant comprising an inflatable bladder, and the electronic assembly further comprises:
an end cap enclosing the reservoir body, where the end cap comprises a tube adapted to fluidically couple the inflatable bladder with the reservoir body.

An aspect of the twenty second embodiment includes wherein the implantable prosthesis comprises an artificial urinary sphincter comprising an inflatable cuff, and the electronic assembly further comprises:
an end cap enclosing the reservoir body, where the end cap comprises a tube adapted to fluidically couple the inflatable cuff with the reservoir body.

An aspect of the twenty second embodiment includes wherein the electronic assembly comprises a cover secured to the canister;
wherein the cover comprises a channel adapted to allow the liquid in the reservoir body to enter the cover and surround the motor.

An aspect of the twenty second embodiment includes wherein the motor comprises a shaft coupled with the pump, with the motor and the shaft adapted to rotate in the liquid to obviate sealing the shaft from the liquid.

An aspect of the twenty second embodiment includes wherein the valve assembly comprises:
a valve body having a suction housing and a discharge housing;
a membrane positioned between the suction housing and the discharge housing;
a first poppet positioned between the membrane and the suction housing; and
a second poppet positioned between the membrane and the discharge housing;
wherein the suction housing is fluidly coupled to the reservoir body and the discharge housing is fluidly coupled to an inflatable bladder of the implantable prosthesis.

An aspect of the twenty second embodiment includes wherein the electronic assembly comprises a cover secured to the canister, and the cover comprises a channel adapted to allow the liquid in the reservoir body to enter the cover and contact the valve assembly;
wherein the valve assembly comprises a valve body provided with a bypass pathway;
wherein the bypass pathway is configured to continuously allow a portion of the liquid in the inflatable bladder to flow to the reservoir body.

An aspect of the twenty second embodiment includes wherein the implantable prosthesis comprises a pair of inflatable bladders implantable into a penis and the valve assembly comprises a bypass pathway formed in a body of the valve assembly, with the bypass pathway fluidly communicating between the pair of inflatable bladders and the reservoir body, with the bypass pathway configured to continuously allow a portion of the liquid in the pair of inflatable bladders to flow to the reservoir body.

An aspect of the twenty second embodiment includes wherein the implantable prosthesis comprises a pair of inflatable bladders implantable into a penis and the pump assembly comprises a pressure sensor adapted to monitor a pressure in the pair of inflatable bladders, with the motor is adapted to run when the pressure sensor senses a pressure in the pair of inflatable bladders below a set pressure for the pair of inflatable bladders.

An aspect of the twenty second embodiment includes wherein the motor comprises a magnet, and when in a presence of a magnetic field of a magnetic resonance imaging machine, the magnet of the motor creates an induced force, and a combined weight of the reservoir assembly is greater than the induced force to allow the body implantable reservoir assembly to be MRI compatible in a magnetic field having a magnetic flux density in a range from 0.5 T to 1.5 T.

An aspect of the twenty second embodiment further includes:
an end cap sealed to the reservoir body to enclose the electronic assembly inside the end cap and the reservoir body; and
a septum formed in the end cap;
wherein the septum is adapted to self-seal after a needle passes through the septum to deliver an initial amount of liquid to lubricate the motor upon start-up.

An aspect of the twenty second embodiment further includes:
an end cap sealed to the reservoir body to enclose the electronic assembly inside the end cap and the reservoir body; and
an implantable antenna coupled to the end cap and electrically connected to the battery.

An aspect of the twenty second embodiment includes the reservoir assembly of the twenty second embodiment provided as a part of an electronic inflatable penile prosthesis (eIPP), the reservoir assembly comprising an end cap sealed to the reservoir body to enclose the electronic assembly inside the end cap and the reservoir body, and the eIPP comprising:
an implantable antenna coupled to the end cap and electrically connected to the battery;
a main tubing having a first end coupled to the end cap and a second end formed as a junction splitting the main tubing into pair of tubes; and
a pair of inflatable bladders implantable into a penis, with each one of the pair of inflatable bladders coupled with one of the pair of tubes;
wherein the motor operates in a first direction to fill the pair of inflatable bladders with the liquid and a second direction to drain the liquid from the pair of inflatable bladders into the reservoir body.

An aspect of the eIPP of the twenty second embodiment above includes wherein the motor comprises a permanent magnet and a combined weight of the reservoir assembly including the motor and the liquid and the battery and the circuit board configures the eIPP to be MRI compatible in a magnetic field having a magnetic flux density in a range from 0.5 T to 1.5 T.

### Detailed Description

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with reference to the orientation of the Figure(s) being described. Because components of embodiments can be positioned in different orientations, the directional terminology is used for purposes of illustration and is in no way limiting. It is to be understood that other embodiments may be utilized, and structural or logical changes may be made without departing from the scope of the present invention. The following detailed description, therefore, is not to be taken in a limiting sense.

The features of the various exemplary embodiments described in this application may be combined with each other, unless specifically noted otherwise.

The term "proximal" as employed in this application means that part that is oriented closest to a center of the human body. A proximal end is the nearest endmost location of a proximal portion of the thing being described. In one example, the proximal end of the rear tip extender, or the proximal end of the penile prosthesis if no rear tip extender is used, engages with the crus penis.

The term "distal" as employed in this application means that part that is situated farthest away from the center of the human body. A distal end is the furthest endmost location of a distal portion of a thing being described.

As relates to penile implants, a proximal portion of the penile implant is implanted in a vicinity of a descending ramus of the pelvis, and a distal portion of the penile implant is implanted in the pendulous (external portion) of the penis.

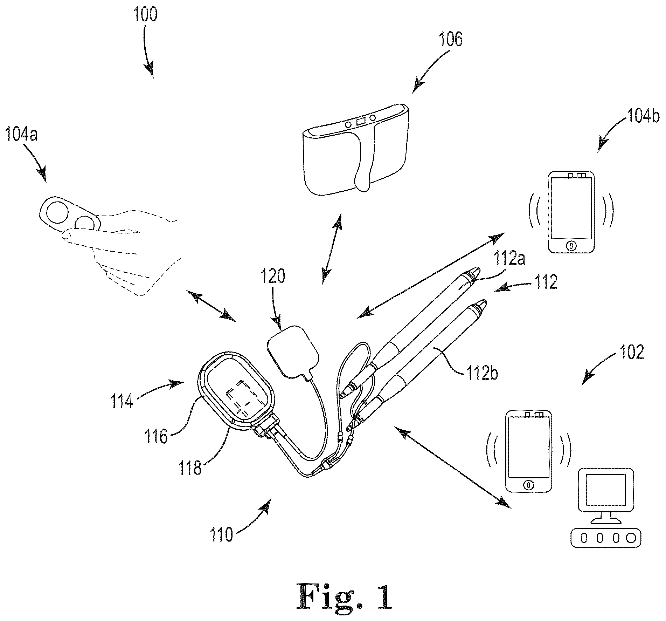

Fig. 1 is a schematic view of an electronic system 100 to treat erectile dysfunction. The electronic system 100 includes an implant 110, a patient interface 104 to control the implant 110, and a recharge assembly 106. It is desirable for the patient, or for the patient and the doctor, to have input and control of the implant 110 when treating erectile dysfunction. The electronic system 100 has several control options, shown in double arrows, including a physician interface 102 that communicates wirelessly with the implant 110 and the patient interface 104 that communicates wirelessly with the implant 110. The patient interface 104 can be a remote activating fob 104a (fob 104a) or a handheld activating device 104b such as an app executable on a smart phone. The recharge assembly 106 can wirelessly recharge a rechargeable battery of the implant 110. The recharge assembly 106 is optional if the implant 110 has a permanent cell battery.

One embodiment of the implant for treating erectile dysfunction is an electronic inflatable penile prosthesis 110 (eIPP 110) including an inflatable penile implant 112 implantable in the penis, a reservoir assembly 114 including a reservoir body 116 adapted to contain a volume of liquid, and an electronic assembly 118 sealed inside of the reservoir body 116. The inflatable penile implant 112 includes a first inflatable bladder 112a implantable into a first corpus cavernosum of the penis and a second inflatable bladder 112b implantable into a second corpus cavernosum of the penis. If the electronic assembly 118 is provided with a rechargeable battery, the eIPP 110 includes an antenna assembly 120 adapted to wirelessly communicate with the recharge assembly 106 to add electrical charge to the rechargeable battery.

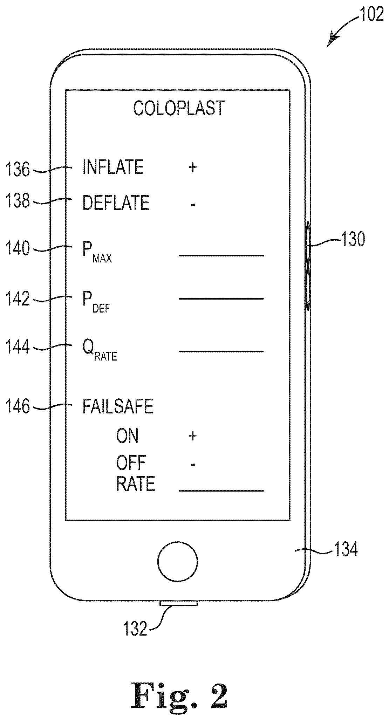

Fig. 2 is a schematic view of a display of the physician interface 102. With reference to Figs. 1-2, the physician interface 102 allows the physician to communicate with and adjust parameters of the eIPP 110, for example immediately after implantation of the eIPP 110 or in subsequent office visits. The physician interface 102 includes a power button 130, a recharge port 132, and an interface screen 134, which can be displayed on a touch screen 134. In addition to other possible physician adjustable parameters, in one embodiment the physician interface 102 includes a control 136 for initiating inflation of the penile implant 112, a control 138 for a rate of deflation or deflation of the penile implant 112, a control 140 allowing the physician to specify a pressure ceiling or a maximum desired pressure Pmax for inflation of the penile implant 112 (for example, a pressure maximum of 20 psi-g), a control 142 allowing the physician to specify a pressure range, a floor, or a specific pressure Pdef for the penile implant 112 when flaccid, a control 144 allowing the physician to specify a flow rate range or the rate of change of liquid flow Qrate for the penile implant 112 during inflation (for example, 2 ml per second), and an icon 146 allowing the physician to override the failsafe leak. The failsafe leak operates to deflate the penile implant 112 through a mechanical orifice if the system 100 loses power or becomes inoperable. During operation, liquid is pumped into the penile implant 112 until the desired set pressure is reached. The icon 146 allows the physician to specify a pressure drop allowed from within the inflatable bladders due to the failsafe leak before the motor is started to top-off the pressure to ensure that the penile implant 112 is maintained at the set pressure selected by the patient during use.

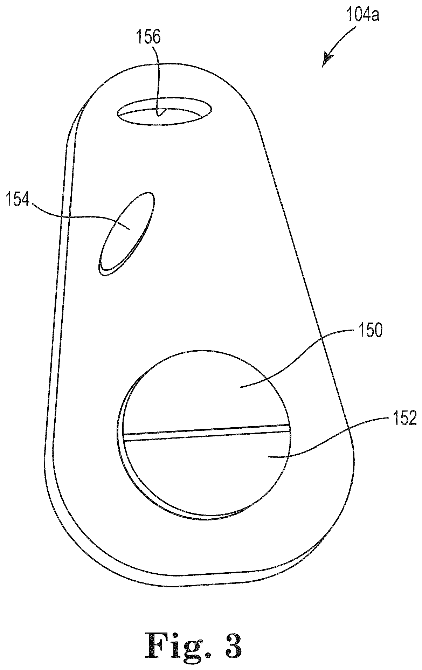

Fig. 3 is a perspective view of the patient interface 104 provided as a remote activating fob 104a (fob 104a). With reference to Fig. 1 and Fig. 3, the fob 104a is an electronic fob that wirelessly communicates with the electronic assembly 118 of the eIPP 110, or the fob 104a communicates with the antenna 120, which in turn communicates with the electronic assembly 118. The fob 104a, external to the patient, could include a pressure sensor that provides a reference pressure measurement, and the pressure in the eIPP 110 is controlled in reference to the pressure measured, or associated with, the fob 104a. The fob 104a is powered by an internal battery (not shown) that energizes a transmitter, where the transmitter emits wireless energy in the form of an electromagnetic wave, for example, as a modulated radio wave, or an infrared pulse received by the eIPP 110. Responsive to receiving input from the fob 104a, the eIPP 110 operates to inflate the penile implant 112, deflate the penile implant 112, turn off, turn on, or disengage the electronics of the eIPP 110. In one embodiment, the fob 104a is adapted to be wirelessly updated with the latest versions of firmware or software. In some embodiments, the patient interface 104 includes an additional applications interface provided by the user's smart phone 104b. In this case, the fob 104a communicates with the smart phone 104b and both the fob 104a and the smart phone app or interface 104b can instruct the eIPP 110 to inflate / deflate the penile implant 112 or turn on or turn off the eIPP 110. One embodiment of the fob 104a includes an inflate touch pad 150, a deflate touch pad 152, and a system-off touch pad 154. A keyhole slot 156 is provided to allow the user to connect the fob 104a to a bracelet, a necklace, a keychain, or the like.

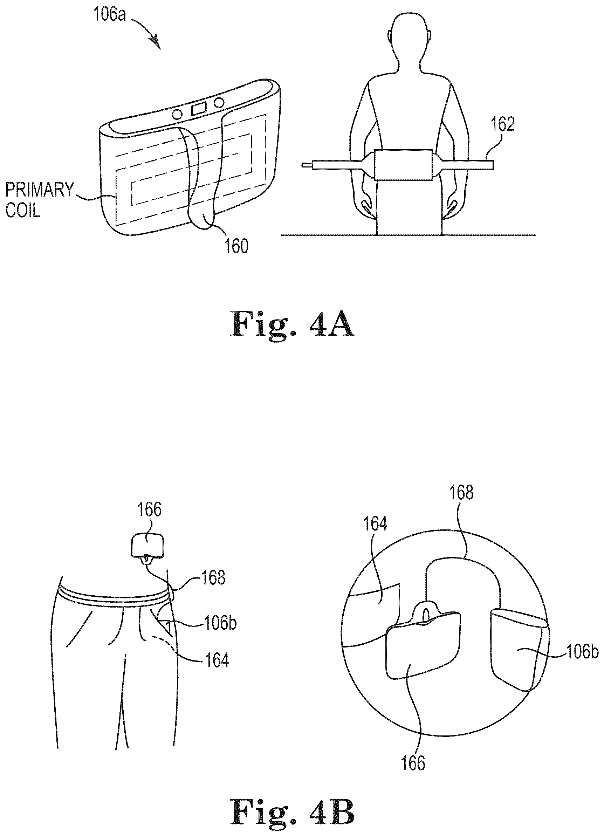

Fig. 4A is a schematic view of one embodiment of a recharge system 106a, and Fig. 4B is a schematic view of another embodiment of a body-worn external recharge system 106b. With reference to Fig. 1, each recharge system 106a, 106b is typically worn or handled by the end-user patient for a short period of time during which energy is transferred from the recharge system 106a, 106b to an implanted coil of the antenna 120 that ultimately recharges (adds electric charge to) a cell or a battery of the electronic assembly 118. Each of the recharge systems 106a, 106b wirelessly couples with the implanted antenna assembly 120 to transmit energy from a primary coil of the recharge system 106a, 106b to a secondary coil of the implanted antenna assembly 120. The energy received by the secondary coil of the antenna assembly 120 is transferred to the battery of the electronic assembly 118 by an electrical coupling. Each of the recharge systems 106a, 106b includes a primary coil adapted to communicate with a secondary coil within the implanted antenna assembly 120. When an alternating current is passed through the primary coil, it creates an oscillating magnetic field. A voltage is induced in the secondary coil when the oscillating magnetic field created by the primary coil is brought into range of the secondary coil in the antenna assembly 120. The induced voltage in the secondary coil can be rectified and regulated to charge a rechargeable battery in the electronic assembly 118 of the eIPP 110.

The embodiment of the recharge system 106a of Fig. 4A includes an internal primary coil (annotated in Fig. 4B) and a keeper 160 that secures the recharge system 106a to a user's belt or waist band. Optionally, the patient is provided with a separate "recharging" belt 162 adapted to secure the recharge system 106a and sized or adjustable to be placed around a waist of the user to align the internal primary coil with the secondary coil of the antenna 120.

The embodiment of the recharge system 106b of Fig. 4B is configured to allow the patient to discretely place the recharge system 106b in a pocket 164 of the user and extend a primary coil 166 secured to the recharge system 106b by a tether 168 to a location on the patient's body that aligns with the secondary coil of the antenna 120. The pocket 164 could be a removable pocket attachable to the patient's trousers, where the pocket 164 is sized to comfortably accept the recharge system 106b. The tether 168 is electrically attached to the recharge system 106b to allow the primary coil 166 to extend a distance away from the recharge system 106, which is discretely and comfortably retained in the pocket 164. The tether 168 ensures that the primary coil 166 of the recharge system 106b can be positioned to optimally align with the secondary coil in the implanted antenna assembly 120. During the recharge period, the recharge system 106b is retained in the pocket 164 of the user and the primary coil 166 extends away from the recharge system 106 for placement or alignment over a skin location under which the implanted antenna 120 is located. This embodiment allows a portion of the recharge system 106b to be discretely maintained out of sight within the pocket 164 while allowing primary coil 166 to extend from the pocket 164, preferably discretely placed under the clothing, to the location of the implanted antenna 120. The primary coil 166 can be referred to as a transmitter coil or transmit coil.

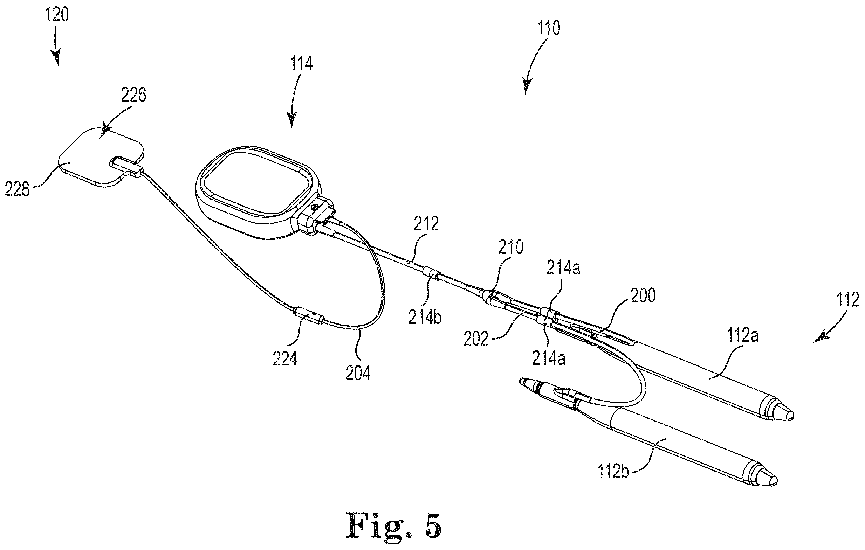

Fig. 5 is a perspective view of one embodiment the eIPP 110. The eIPP 110 is typically provided to a hospital or a surgeon in sterile packaging with its component parts separated. The inflatable penile implant 112 is provided in a range of lengths from about 14 cm to 22 cm. The surgeon selects a desired length for the inflatable penile implant 112 and connects the components intraoperatively, including the inflatable penile implant 112, the reservoir assembly 114, and the antenna assembly 120. Alternatively, the eIPP 110 could be provided to the hospital or the surgeon in an assembled format with the component parts pre-connected.

The eIPP 110 includes the first inflatable bladder 112a coupled by first tubing 200 to the reservoir assembly 114, the second inflatable bladder 112b coupled by second tubing 202 to the reservoir assembly 114, and the antenna assembly 120 coupled by an electrical cable 204 to the reservoir assembly 114. It is advantageous to the surgeon to have flexibility in adjusting lengths of the tubing 200, 202 and a length of the electrical cable 204. In one embodiment, each of the first and second tubing 200, 202 is provided with a connector 214a located between a respective one of the inflatable bladder 112a, 112b and a junction 210 that fluidly joins the two portions of the tubing 200, 202 into a single main tubing 212. The connector 214a allows the surgeon to selectively size the tubing 200, 202 to the patient's anatomy by cutting the tubing 200, 202 to a desired length and reattaching the severed portions of the tubing 200, 202 with the connector 214a. An alternative option is provided by a connector 214b that allows the surgeon to cut the main tubing 212 to a desired length to adjust a distance between the junction 210 and the reservoir assembly 114. Each of the connectors 214a, 214b provides a fluidic coupling between the two cut ends of the tubing 200, 202 or the main tubing 212. The tubing 200, 202, 212 is a kink-resistant tubing that can be bent without collapsing the lumen inside the tube. One example of kink-resistant tubing is provided by a tubular body having a lumen with a nylon strand wound over the tubular body and overcoated in polymer, where the polymer is suitably selected from silicone, polybutylene, or block co-polymers of butylene, as examples.

The reservoir assembly 114 includes the reservoir body 116 that is sized to contain some of the liquid of the device and the electronic assembly 118. The reservoir assembly 114 is body implantable, for example within the abdomen, the thorax, or the pelvis, with a preferred location in the abdomen. The electronic assembly 118 inside the reservoir body 116 is fluidically coupled to the penile implant 112 by the main tubing 212 and electrically coupled to the antenna assembly 120 by the electrical cable 204. An electrical connector 224 is provided to electrically couple the antenna assembly 120 to the reservoir assembly 114 after implantation of these two components of the eIPP 110.

The antenna assembly 120 includes a receiver coil 226, which is sometimes referred to as a secondary coil 226 or the receive coil. In one embodiment, the receiver coil 226 is a multilayer assembly provided with electrical windings that are not shown as they are under a cover 228. The cover 228 is a polymer cover, with one suitable cover being silicone. Another suitable cover 228 for the antenna 120 is a ceramic covering or coating over the electrical coil windings. The receiver coil 226 is body-implantable, for example within the abdomen, the thorax, or the pelvis, with a preferred location in one of the abdomen or the thorax. There can be advantages to implanting the antenna assembly 120 at a location spaced apart from the reservoir assembly 114. For example, in cases where the antenna assembly 120 is employed to charge or recharge a battery of the eIPP 110, it can be beneficial to implant the antenna assembly 120 at a location that allows for discreet charging of the battery (for example, near the beltline of the user). The reservoir assembly 114 can be placed posterior the muscle or anterior the muscle and under the fascia, depending on surgeon preference. Placement of the reservoir assembly 114 sub-muscularly can be more comfortable for the patient. It can be beneficial to position the antenna closer to the skin surface for improved coupling with the external charging coil. This improves the induction between the charging coil and the antenna, so power can be transferred more efficiently. Specifically, positioning the antenna closer to the skin surface (for example, less than 2 cm deep) for improved coupling with the external charging coil increases a coupling coefficient between the charging coil and the antenna so power may be transferred more efficiently, allowing higher power to be delivered. For cases where the antenna assembly 120 is implanted near the beltline of the user, the reservoir assembly 114 is implanted in a different location, for example within the abdomen or even within the thorax.

The windings of the receiver coil 226 are configured to wirelessly interact with the external recharge system 106 (Fig. 1 and Figs. 4A-4B) to provide wireless power transfer from the recharge system 106 to the power source of the eIPP 110. In an exemplary embodiment, the recharge system 106 provides a first primary coil, and the receiver coil 226 provides a secondary implant coil. An alternating magnetic field is formed in the primary coil when an alternating current flows through the primary coil. A voltage will be induced in the secondary coil when the secondary implant coil of the receiver coil 226 is within range of the alternating magnetic field of the primary coil. The induced voltage is rectified and regulated to charge the power source of the eIPP 110 or to directly power the eIPP 110. The primary (external) and secondary (implantable) coils are tuned to resonate at the same frequency, the frequency of which is selected / balanced to reduce tissue heating while increasing the efficiency of the wireless power transfer.

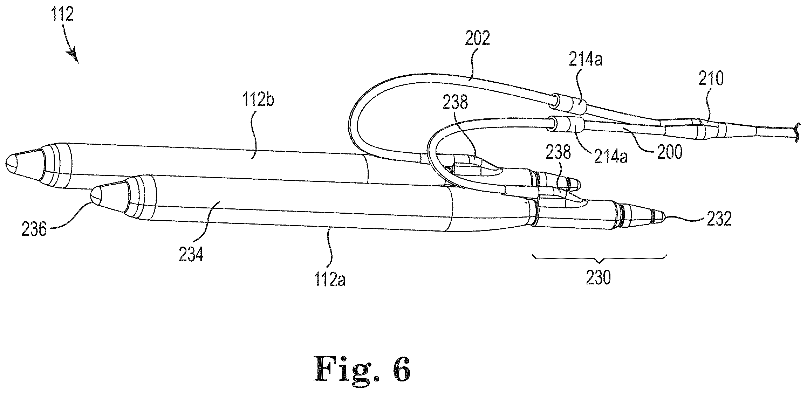

Fig. 6 is a perspective view of one embodiment of the inflatable penile implant 112. Each of the first inflatable bladder 112a and the second inflatable bladder 112b includes a proximal end portion 230 having a proximal end 232, an inflatable bladder 234 having a distal end 236, and a tubing housing 238 coupled to the proximal end portion 230. The tubing 200 or tubing 202 is integrated with the tubing housing 238 such that the tubing 200, 202 lies flat against, or is nearly parallel with, the inflatable bladder 234. The tubing housing 238 provides a strain relief that resists forces applied to the inflatable bladder 112a when the tubing 200 is pulled or stressed, either during implantation or during use. During implantation, the proximal end portion 230 (also called a rear tip) is implanted in the crus of the penis located along each descending ramus of the pelvis and the distal end 236 is implanted within the glans penis of the external penis.

The proximal end portion 230 of the inflatable bladder 112a, 112b is provided as a solid mass of polymer, for example polyurethane or silicone. The inflatable bladders 234 are like balloons, fabricated from soft polymer material that collapses when the inflatable bladder 234 is emptied of liquid, providing the penis with a flaccid state, and expands like a balloon when the inflatable bladder 234 is inflated with liquid, providing the penis with an erect state. The inflatable bladders 234 are illustrated in an inflated state to show an expanded girth. Suitable material for fabricating the inflatable bladder 112 includes silicone or other biocompatible polymers with examples including co-polymers, block co-polymers, blends of polymers in general, or urethanes, or co-polymers, block co-polymers, or blends of polymers with urethane, or the like. At least the inflatable bladders 234 are coated with a hydrophilic coating that becomes lubricious when wetted with an aqueous solution, the lubricity of which aids in inserting the inflatable bladder 112a, 112b into the corpora cavernosa. In one embodiment, the entirety of the eIPP 110 is coated with a hydrophilic coating. Prior to implantation, an antibiotic solution can be added to the aqueous solution, and the hydrophilically coated eIPP 110 is placed in the solution to impart antimicrobial activity to the hydrophilically coated portions of the eIPP 110.

The inflatable bladder 112a, 112b are provided in a range of lengths from 10 cm to 28 cm, with a preferred range of lengths for the inflatable bladders 112a, 112b being between 14 - 22 cm. Suitable inflatable bladder 112a, 112b are available from Coloplast Corp., Minneapolis, Minnesota.

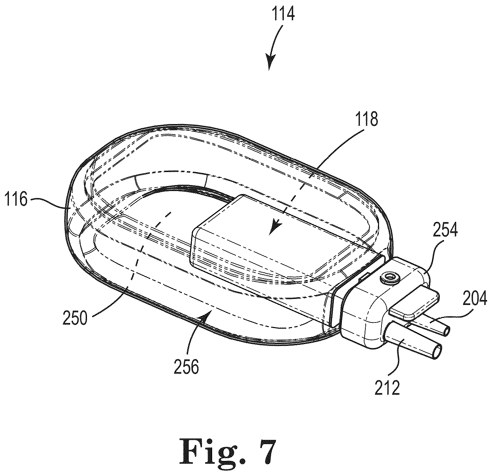

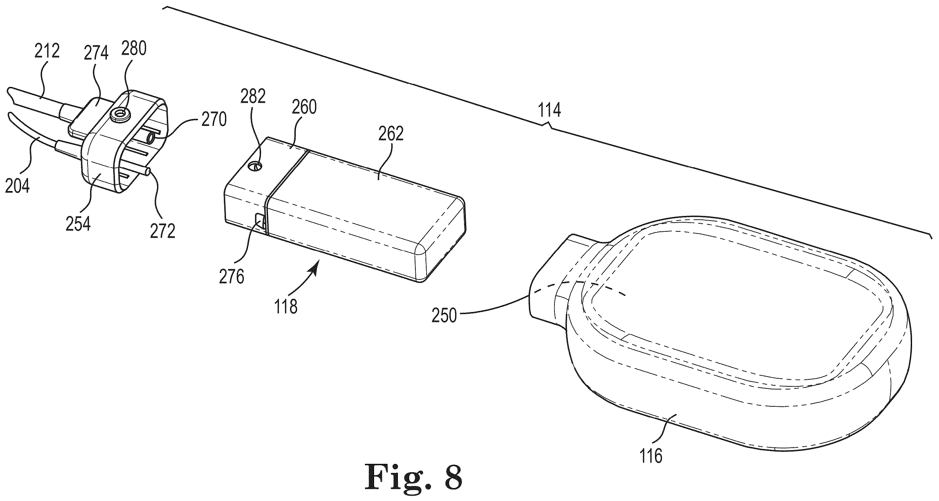

Fig. 7 is a perspective view of the reservoir assembly 114 assembled and Fig. 8 is a perspective view of components of the reservoir assembly 114 disassembled in an exploded view.

The reservoir assembly 114 includes the electronic assembly 118 inserted into the reservoir body 116. The reservoir body 116 is sized to contain a volume of liquid (and the electronic assembly 118), where the liquid is movable from the reservoir body 116 into inflatable penile implant 112 (Fig. 6) through the tubing 212. The electronic assembly 118, through the receiver coil 226, communicates wirelessly with the physician interface 102 (Fig. 2) and / or the patient interface 104 (See Fig. 3) and electrically couples with the recharging system 106 (Figs. 4A-4B) through the antenna assembly 120 (Fig. 5).

The reservoir body 116 defines an internal cavity 250 sized to contain the volume of liquid and the electronic assembly 118. An end cap 254 is connected to the reservoir body 116 to seal the internal cavity 250 and form a liquid-tight sealed enclosure 256, such that, when assembled, the electronic assembly 118 is sealed within the enclosure 256 and resides in the internal cavity 250. In this way, the electronic assembly 118 is immersed in liquid within the reservoir body 116 to provide the reservoir assembly 114 with a compact platform that offers advantages during implantation and contributes to compatibility of the eIPP 110 to magnetic fields from magnetic resonance imaging (MRI) machines.

The reservoir body 116 is sized to contain a volume of liquid in a range from approximately 40 cubic centimeters (cc) to approximately 200 cc, with one preferred volume range for the reservoir body 116 being from about 60 - 120 cc. A suitable liquid for the system is sterile saline at 0.9%. Suitable material for fabricating the reservoir body 116 includes silicone or other biocompatible polymers with examples including co-polymers, block co-polymers, or blends of polymers in general, or urethanes, or co-polymers, block co-polymers, or blends of polymers with urethane, or the like. Suitable material for fabricating end cap 254 includes silicone or other biocompatible polymers with examples including co-polymers, block co-polymers, or blends of polymers in general, or urethanes, or co-polymers, block co-polymers, or blends of polymers with urethane, or the like.

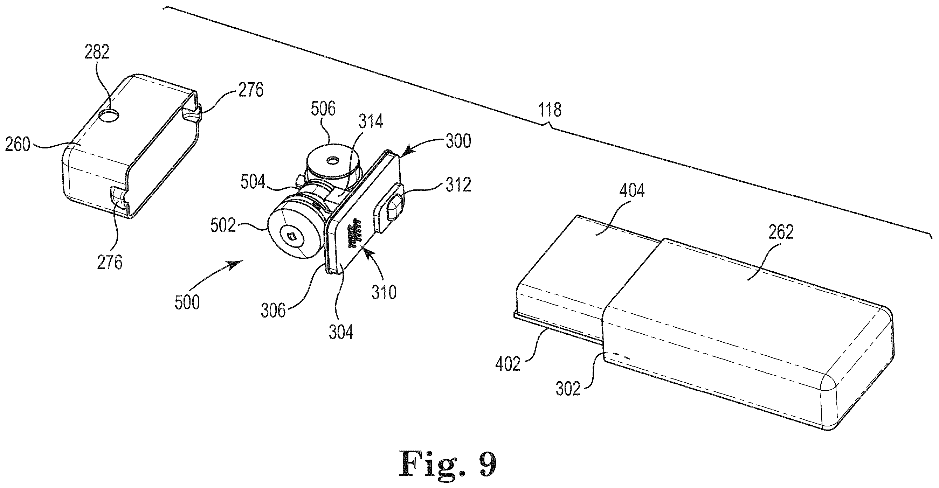

With reference to Figs. 8 and 9, the electronic assembly 118 includes a cover 260 attached to a canister 262. As described in further detail below, the electronic assembly 118 includes a pump assembly 500 that includes a motor 502 that drives a pump 504 and these components are separated from a power source and printed circuit board; the motor 502 and the pump 504 are retained within the cover 260 and the power source and the printed circuit board are hermetically sealed within the canister 262. The cover 260 is connected to the canister 262 or to seal 300, and in one embodiment the cover 260 is a titanium header that is laser welded to a titanium canister 262, although other approaches for securing the cover 260 to the canister 262 while suitable for a body-implantable medical device are also acceptable. The cover 260 allows liquid to enter and wet or interact with the motor 502 and the pump 504.

The end cap 254 includes a first passage 270 that fluidically connects the tubing 212 and the inflatable penile implant 112 (Fig. 6) with a valve assembly 506 and the pump 504 inside of the cover 260, and a second passage 272 for electrical connection between the antenna assembly 120 (Fig. 5) and the power source and printed circuit board inside of the canister 262. In one embodiment, the end cap 254 is formed or molded from silicone or other biocompatible polymers, with examples including co-polymers, block co-polymers, or blends of polymers in general, or urethanes, or co-polymers, block co-polymers, or blends of polymers with urethane, or the like. The end cap 254 includes a gripping tab 274 that provides a solid location for gripping with a surgical tool, which is useful during implantation or removal of the reservoir assembly 114 from the patient.

One advantage of the disclosed reservoir assembly 114 is that the electronic assembly 118 is immersed in the liquid within the reservoir body 116, where the motor 502 and the pump 504 of the electronic assembly 118 communicate with the liquid within the reservoir body 116 through a channel 276 or louvre 276 formed in the cover 260. The channel 276 allows the liquid in the reservoir body 116 to freely enter the cover 260 and surround the motor 502 and the pump 504, described below, that are retained inside of the cover 260. The canister 262 is hermetically sealed to prevent the liquid in the reservoir body 116 or in the cover 260 from entering the canister 262 and affecting the enclosed circuit board and electronics. One of the benefits of this approach is that the motor is both cooled and lubricated by the liquid in the reservoir body 116. Another benefit of this approach is that the shaft of the motor 502 (retained inside of the cover 260) need not include a seal that separates the fluid from any part of the motor 502 or the electronics that control the motor 502. Rather, the motor 502 is immersed in the liquid in the reservoir body 116 and operates more efficiently without a liquid-tight seal for the shaft.

When the reservoir assembly 118 is first implanted in a patient it might be the case that the liquid to be introduced to the reservoir body 116 has not yet made contact with the motor 502 and the pump 504 components insider of the cover 260, which could result in the motor 502 / pump 504 not being lubricated by liquid and thus having an undesirable initial startup in dry conditions. A void formed in the end cap 254 and a septum passage 282 located on the reservoir body 116 are provided to allow a surgeon to introduce an amount of liquid, for example sterile saline provided in a syringe, through the end cap 254 and then through the septum passage 282 formed in the cover 260 to pre-lubricate or lubricate the motor 502 / pump 504 to reduce or avoid high-friction startup of a non-lubricated motor / pump 504. A threaded plug 280 can be removed and installed in the void in the end cap 254. Alternatively, the septum can connect to a fluid branch (not shown) that connects to the suction or discharge conduit of the pump, thereby directly lubricating the pump.

Referring to Fig. 9, the electronic assembly 118 is disassembled to show its components. The electronic assembly 118 includes a seal 300 attachable to the canister 262 to hermetically seal a printed circuit board (PCB) 402 and a battery 404 inside an enclosure 302, the pump assembly 500 supported by the seal 300 and in electrical communication with the PCB 402 and the battery 404, and the cover 260 provided to contain the pump assembly 500, where the cover 260 is connected to the canister 262 as described above.

The seal 300 has a first side 304 that seals with the canister 262 and an opposite second side 306 that supports the pump assembly 500. The seal 300 ensures that the enclosure 302 inside of the canister 262 is hermetically sealed. The seal 300 includes an electrical feedthrough 310 bridging across the first side 304 to the second side 306 that forms a hermetically sealed passage for an electrical connection between the pump assembly 500, the PCB 402, and the battery 404. In one embodiment, the feedthrough 310 is a ten-pin connector adapted to connect an electrical motor of the pump assembly 500 with the PCB 402.

A pressure sensor 312 is disposed on the first side 304 of the seal 300. The pressure sensor 312 includes a port formed through the seal 300, where the port (shown and described below) is in fluid communication with the main tubing 212 and the inflatable penile implant 112 (See Fig. 5) through pump assembly 500. The port allows the pressure sensor 312 to register a pressure in the inflatable penile implant 112 that is electrically communicated to the controller of the PCB 402. The controller of the PCB 402, cooperating with the pressure sensor 312, monitors the sensed pressure in the inflatable penile implant 112 to control when the sensed pressure equals the desired set pressure to thus turn off the pump assembly 500 and stop inflation of the inflatable penile implant 112, or to operate the pump assembly 500 to add liquid to the inflatable penile implant 112 to achieve the desired set pressure.

One embodiment of the seal 300 is a ceramic seal 300 that is connected to a titanium canister 262, where suitable ceramic seals include metal-ceramics such as alumina (both crystalline or polycrystalline), zirconia such as magnesium oxide stabilized zirconia, or yttria-stabilized polycrystals. One suitable connection between the ceramic seal 300 and the canister 262 is a brazed connection, where one suitable braze material is gold. One exemplary hermetically sealed enclosure 302 is provided by gold brazing an alumina ceramic seal 300 to a titanium canister 262. Another embodiment is a titanium seal 300 that is welded to titanium cannister 262.

In one embodiment, the battery 404 is a secondary, or rechargeable, lithium battery having a voltage in a range from 2.5 - 5.0 volts, a capacity in a range from 200 - 500 mAh, and a mass in a range from about 15 - 25 g. One suitable battery is a Contego 440 medical power battery available from Resonetics, Nashua, NH, having a voltage in a range from 3.0 - 4.1 V, a capacity of 440 mAh, and a mass of 19.1 g. The battery 404 is electrically coupled to the antenna assembly 120 (Fig. 5) through, for example, a battery management circuit on the PCB 402, where the antenna assembly 120 is adapted to wirelessly couple with an external source to add charge to the battery 404, which results in recharging the battery 404.

With reference to Fig. 5 - Fig. 9, the pump assembly 500 is operable to: A) move liquid into and pressurize the inflatable bladders 112a, 112b of the penile implant 112 to a pressure in a range from about 10 pounds-per-square-inch (psi-g) to about 20 psi-g; B) suitably inflate empty inflatable bladders 112a, 112b to a pressure above 15 psi-g in less than 120 seconds for a 24 cm length inflatable bladder 112a,b; C) provide a flow rate of liquid to and from the inflatable bladders 112a,b from about 100 ml/min at zero psi to about 50 ml/min at 20 psi-g; and move liquid out of the inflatable bladders 112a, 112b and into the reservoir body 116.

Suitable liquids for the eIPP 110 include saline at 0.9%, a dextrose solution, saline in solution with chlorhexidine gluconate, or a dextrose solution with chlorhexidine gluconate. A preferred working liquid for the eIPP 110 is a saline solution.

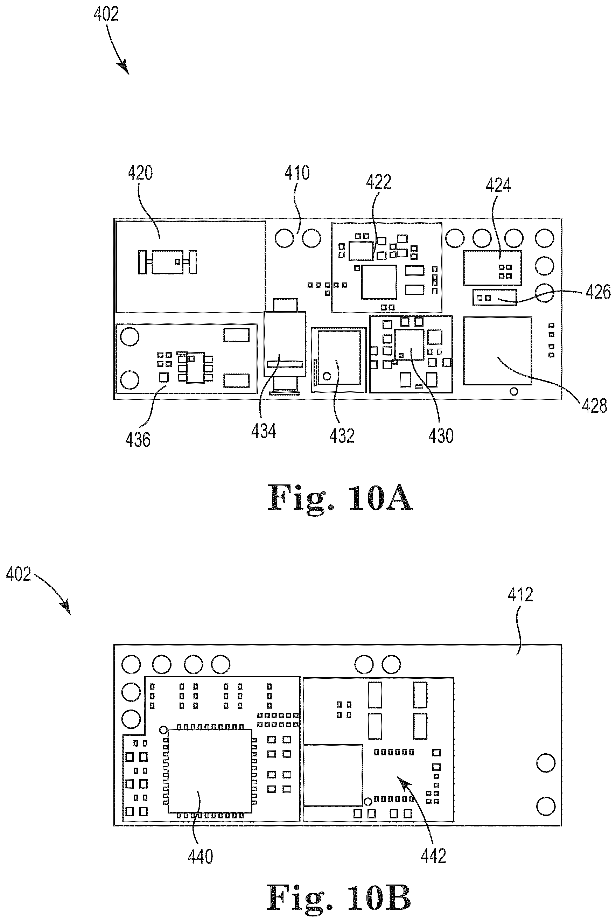

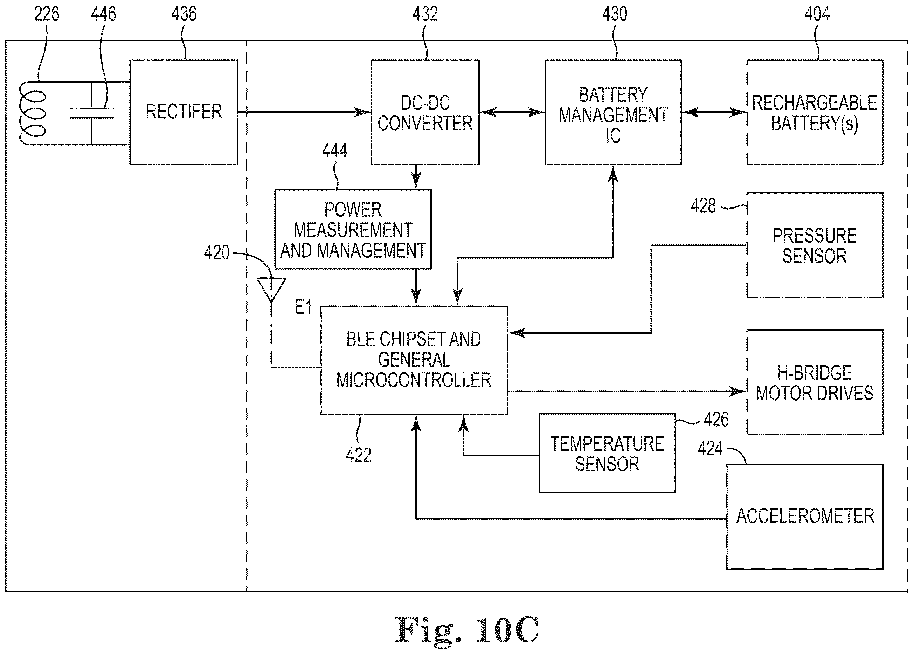

Fig. 10A is a top view and Fig. 10B is a bottom view of the PCB 402. Fig. 10C is an architectural schematic of the PCB 402. Referring to Figs. 10A and 10B, the PCB 402 has a top surface 410 opposite a bottom surface 412.

Referring to Figs. 10A-10C, the top surface 410 of the PCB 402 is populated with several chipsets which include, as one example, an antenna 420, a Bluetooth Low Energy (BLE) chipset 422, an accelerometer 424, a temperature sensor 426, a pressure sensor 428, a battery charger 430, a regulator module 432, a Zener diode 434, and a rectifier 436. The bottom surface 412 of the PCB 402 is populated with several chipsets which include, as one example, a brushless direct current (BLDC) control 440 and a BLDC converter 442.

The receiver coil 226 is coupled to the PCB 420. The antenna 420 can be formed from copper. The receiver coil 226 can be formed from copper traces on a substrate such as a printed circuit board. The copper wire can be mounted on a printed circuit board. The receiver coil 226 can be formed from enameled copper wire or Litz wire wound around a former. The receiver coil 226 can be generally co-located with the rectifier 436. The leads of the coil can be used to deliver power to the rectifier 436 to improve system electrical efficiency. The coil can be encapsulated in a biocompatible material such as silicone rubber.

The PCB 402 can include a power management system 444 including power regulation circuitry, power measurement circuitry, the battery 404, and the battery charger 430 with battery charger management electronics. The power measurement circuitry of the power management system 444 can include the battery charger 430, the regulator module 432, the Zener voltage clamp 434, the rectifier 436, and the BLDC converter 442. The power management system 444 can adjust settings to accommodate a range of coupling conditions between the transmit coil and the receive coil (the antenna 420).

Under some transmit coil 160 and receive coil 226 coupling conditions, such as when a greater than normal distance is between the transmit coil 160 and the receive coil 226, the transmit coil 160 may be called upon to transmit at significantly higher power levels than if the transmit coil 160 and the receive coil 226 are more closely aligned. The power measurement circuitry can include a closed-loop control system to reduce power dissipation within the eIPP 110 when additional transmit power is not required. In other implementations without the closed-loop control system, the eIPP 110 could dissipate additional received power as heat or include a variable tuning network allowing the receive coil 226 to be detuned when the additional power is not needed.

The DC-DC converter 432 converts the variable input voltage from the receive coil 226 to a voltage suitable for charging the battery 404 and powering the other electrical components. The DC-DC converter 432 also powers the electrical components from the battery 404 when no external charging field is present at the receiver coil 226.

The rectifier 436 can include a capacitor 446. The capacitor 446 can be a voltage-controlled capacitor for tuning the receiver coil 226 "on the fly."

The power management system 444 can include the Zener diode 434. The Zener diode 434 can clamp the voltage to prevent circuit damage in the case of a high transmitter output driven with high coupling coefficient. For example, the Zener diode 434 can be a 1 Watt Zener diode, however, another suitable capacity Zener diodes may be used.

The brushless direct current (BLDC) control 440 controls the motor 502 to drive the pump 504. The BLDC control 440 performs control and feedback functions for the motor 502 and can limit maximum power in the motor 502.

The BLDC converter 442 converts the voltage from the battery 404 to an acceptable voltage for the motor 502. For example, in some implementations, the motor 502 can be specified for a 24-volt supply.

In some implementations, the fluid within inflatable bladders 112a, 112b can be manually forced out of the inflatable bladders 112a, 112b and back through the pump 504 to the reservoir assembly 114, causing the pump 504 to rotate in reverse. As a result of the pump 504 operating in reverse, the motor 502 likewise operates in reverse, and electricity can flow from the motor to the battery 404, charging the battery 404. This can be referred to as harvesting energy.

The BLE chipset 422 allows both Bluetooth communications and general control of the eIPP 110. The BLE chipset 422 is connected to the Bluetooth antenna 420 located within the eIPP 110. The antenna 420 can be a PCB antenna with the canister 262 (Fig. 9) being ceramic or another non-metallic material. When the canister 262 is metal, the antenna 420 can be a stamped metal antenna encapsulated into a header using feedthroughs to provide an interface to the PCB 402.

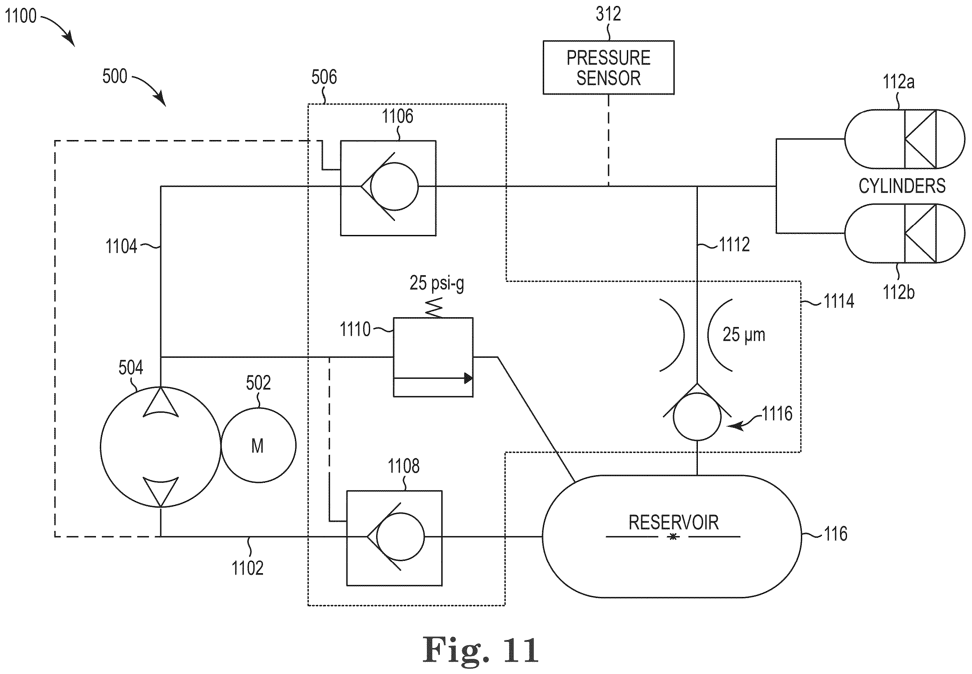

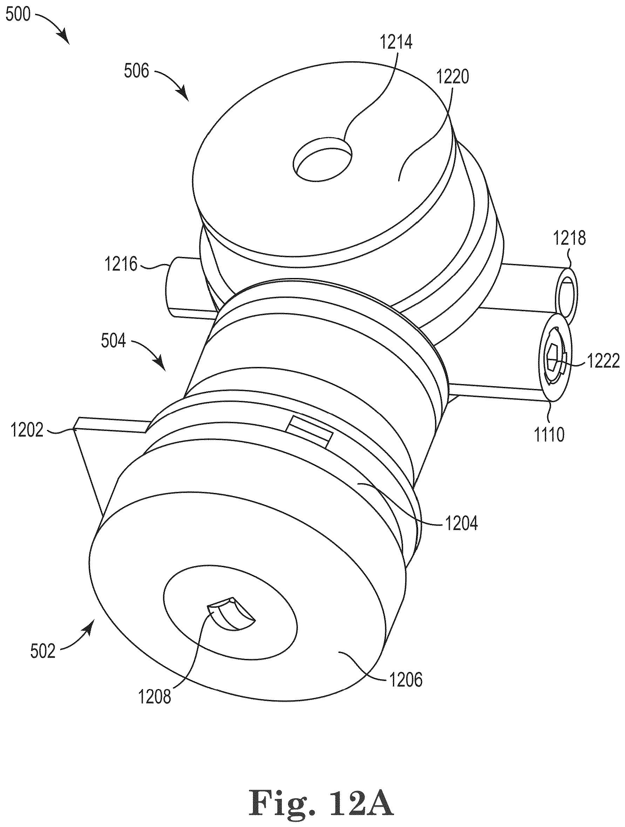

Fig. 11 is a piping and instrumentation diagram that functionally illustrates the components of the eIPP 110 including the reservoir body 116, inflatable bladders 112a, 112b, the pump assembly 500 (the motor 502, the pump 504, and the valve assembly 506), and the pressure sensor 312. The pump assembly 500 is shown and described in more detail in reference to Figs. 12A-12B.

Referring to Fig. 11, the motor 502 is operated responsive to control signals from the controller of the PCB 402. The motor 502 operates to rotate the pump 504 in either a first state or a second state. The pump 504 operating in the first state inflates the inflatable bladders 112a, 112b. When operating in the first state, the pump 504 rotates in a first direction to draw fluid from the reservoir body 116 through conduit 1102 and then supply the fluid to the inflatable bladders 112a, 112b through conduit 1104 to increase a fluid pressure in the inflatable bladders 112a, 112b. The pump 504 operating in the second state deflates the inflatable bladders 112a, 112b. When operating in the second state, the pump 504 rotates in a second direction to draw fluid from the inflatable bladders 112a, 112b through conduit 1104 and then supply the fluid to the reservoir body 116 through conduit 1102 to decrease a fluid pressure in the inflatable bladders 112a, 112b.

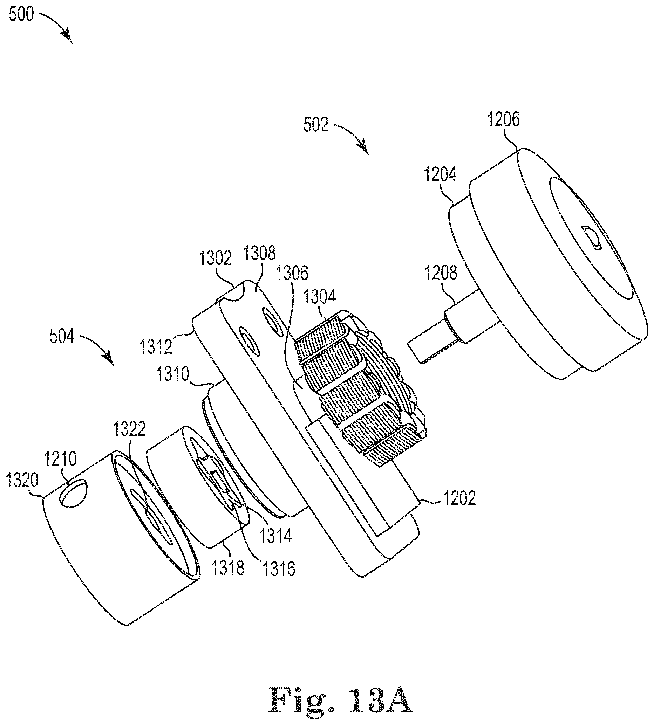

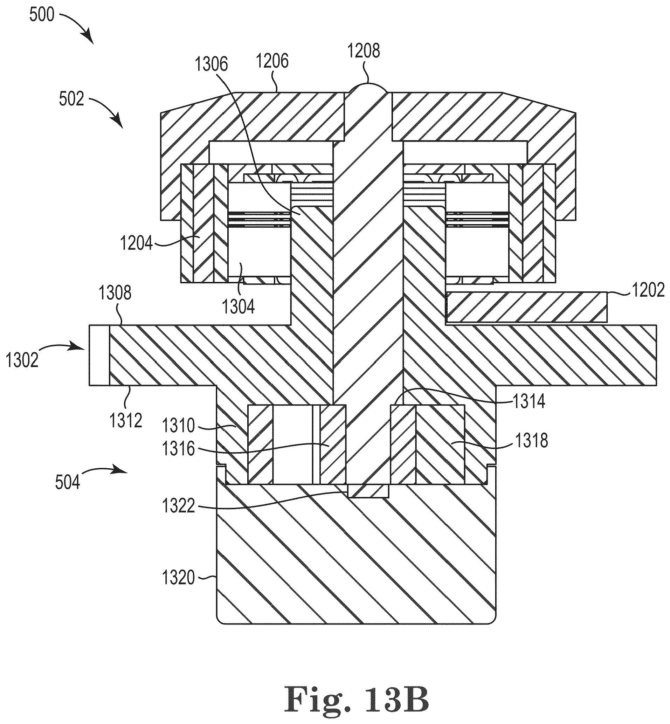

The motor 502 operates the pump 504. The motor 502 is described in more detail in reference to Figs. 12A-12B and 13A-13B.

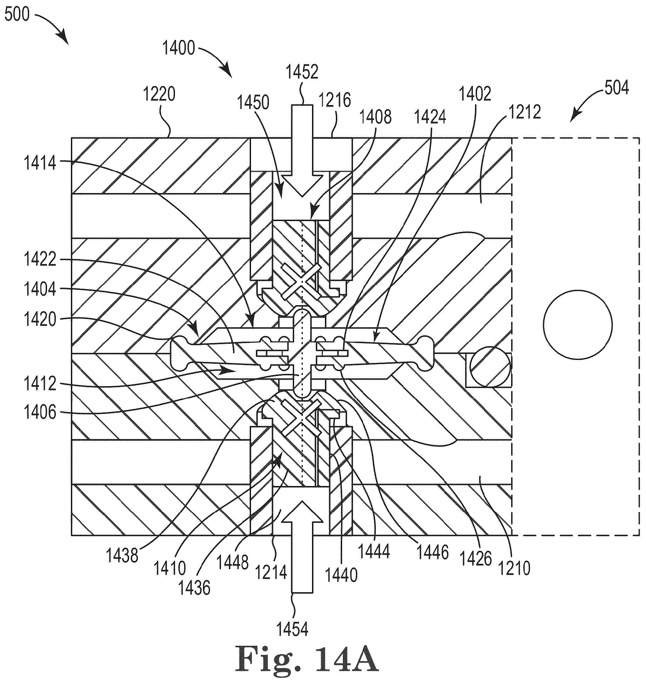

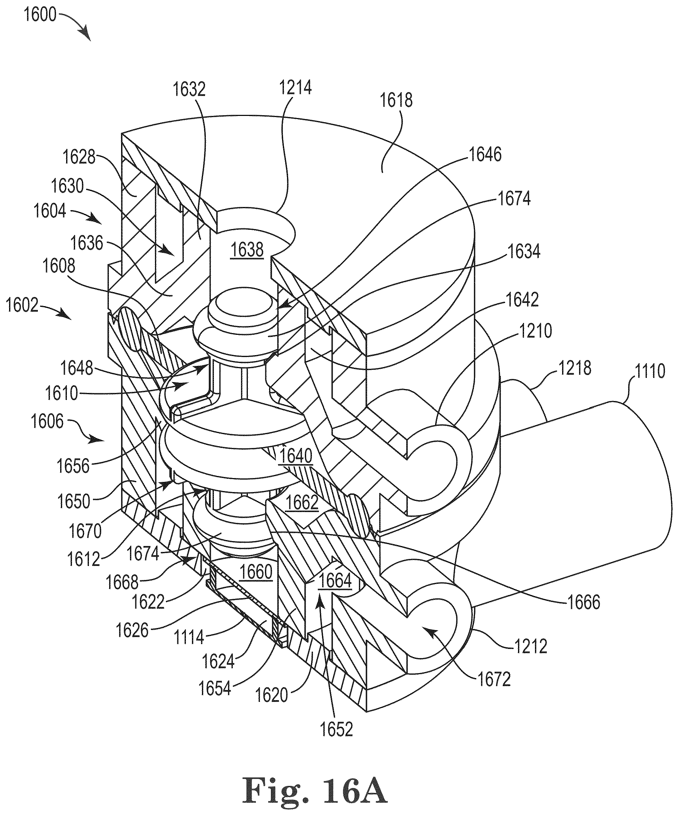

The valve assembly 506 controls fluid flow through the conduit 1102 and the conduit 1104 responsive to operation of the pump 504 by the motor 502 to move fluid between the reservoir body 116 and the inflatable bladders 112a, 112b. A first embodiment of the valve assembly 506 is the first valve assembly 1400. The first valve assembly 1400 is shown and described in more detail in reference to Figs. 14A-14E. A second embodiment of the valve assembly 506 is the second valve assembly 1600. The second valve assembly 1600 is shown and described in more detail in reference to Figs. 16A-16G.

The first valve assembly 1400 (described below in Figs. 14A-14E) controls flow through a first check valve portion 1106, a second check valve assembly 1108, a pressure relief portion 1110, and a bypass pathway 1112 with an orifice 1114 and optionally a check valve 1116.

Referring to Fig. 11, the valve assembly 506 includes the bypass pathway 1112. The bypass pathway 1112 is a mechanical safety to ensure the inflatable bladders 112a, 112b will deflate even if the system becomes inoperable. The bypass pathway 1112 fluidly couples the inflatable bladders 112a, 112b and the reservoir body 116. The bypass pathway 1112 is continually open allowing a portion of the fluid to flow between the inflatable bladders 112a, 112b and the reservoir body 116. During use, the motor 502 operates the pump 504 to top-off or maintain a desired set pressure in the inflatable bladders 112a, 112b, the pressure of which is sensed by the pressure sensor 312 (Fig. 9) and controlled by the controller of the PCB 402. Fluid flows between the inflatable bladders 112a, 112b and the reservoir body 116 based on a pressure differential between the inflatable bladders 112a, 112b and the reservoir body 116.

The bypass pathway 1112 provides a conduit for fluid to flow between the inflatable bladders 112a, 112b and the reservoir body 116. The pump 504 can be operated in a first direction and a second direction. When the pump 504 is operated in the first direction, the fluid flows from the reservoir body 116 to the inflatable bladders 112a, 112b. When the pump 504 is operated in the second direction, the fluid flows from the inflatable bladders 112a, 112b to the reservoir body 116.

When the pump 504 moves fluid out of the reservoir body 116, the inflatable bladders 112a, 112b fill with the fluid and the pressure increases inside the inflatable bladders 112a, 112b. The inflatable bladders 112a, 112b expand in volume, generally filling the corpus cavernosum. When the desired or set pressure is reached, the controller of the PCB 402 shuts off the pump 504. The valve manifold 506 shuts, sealing the fluid in the inflatable bladders 112a, 112b, thereby maintaining the inflatable bladders 112a, 112b in a pressurized state for a period of time. When the user no longer needs to maintain the inflatable bladders 112a, 112b in the pressurized state, the controller of the PCB 402 turns on the pump 504 in the second direction, and the pump moves the fluid from the inflatable bladders 112a, 112b to the reservoir body 116. If the pump 504 becomes inoperable, the inflatable bladders 112a, 112b can deflate based on the pressure differential between the inflatable bladders 112a, 112b and the reservoir body 116 causing fluid from within the inflatable bladders 112a, 112b to flow from the inflatable bladders 112a, 112b through the bypass pathway 1112 back to the reservoir body 116 without passing through the pump 504.

The bypass pathway 1112 is sized to maintain the fluid in the inflatable bladders 112a, 112b for the desired period of time and maintain the pressure within the inflatable bladders 112a, 112b within a normal operating pressure threshold range, while still allowing fluid to flow from the inflatable bladders 112a, 112b to the reservoir body 116. When the inflatable bladders 112a, 112b are pressurized to within the normal operating pressure threshold range and the pump 504 is not operating, the fluid in the inflatable bladders 112a, 112b can flow to the reservoir within a predetermined period of time based on the dimensions of the bypass pathway 1112 and the pressure differential between the inflatable bladders 112a, 112b and the reservoir body 116. For example, the predetermined period of time can be between one and twenty-four hours. In this implementation, the predetermined period of time is approximately six hours. The rate at which the fluid flows from the inflatable bladders 112a, 112b to the reservoir body 116 may cause a slight pressure decrease in the inflatable bladders 112a, 112b during use, however the pressure decrease may be imperceptible by the user before the motor 502 turns on to top-off the inflatable bladders 112a, 112b.

The bypass pathway 1112 has a diameter in a range from 10 to 40 micrometers (µm). In this implementation, the diameter is approximately 25 micrometers (µm). In other implementations, the bypass pathway 1112 can have another suitable diameter.

The bypass pathway 1112 can include an orifice 1114 to restrict flow between the inflatable bladders 112a, 112b and the reservoir body 116. In this implementation, the orifice 1114 has a diameter of 25 micrometers (µm). In other implementations, the orifice 1114 can have another suitable diameter. When the bypass pathway 1112 includes the orifice 1114, the diameter of the bypass pathway 1112 is larger than the diameter of the orifice 1114.

Referring to Figs. 12A-12B, the pump assembly 500 includes the motor 502, the pump 504, and the valve assembly 506. The pump 504 rests in a cradle 314 (Fig. 9) of the ceramic seal 300 (Fig. 9). The cradle 314 extends from the opposite second side 306 of the ceramic seal 300.

The motor 502 is mounted to one side of the pump 504. The valve assembly 506 is mounted on the opposite side of the pump 504 from the motor 502.

Referring to Figs. 12A-12B and 13A-13B, the motor 502 includes a bearing base 1302, a printed circuit board 1202 optionally mounted on the bearing base 1302, a motor rotor 1204 with a rotor hub 1206, a shaft 1208 coupled to the motor rotor 1204 and the pump 504, and a motor stator 1304 (shown in Figs. 13A-13B). The shaft 1208 extends through the motor stator 1304 to couple to the pump 504. The motor rotor 1204 rotates relative to the motor stator 1304 to operate the pump 504. The rotor hub 1206 serves as a cover for the motor 502 and couples the shaft 1208 to the motor rotor 1204. The motor rotor 1204 includes one or more magnets. The motor stator 1304 includes multiple wire windings that receive and conduct electricity relative to the magnets, causing the motor rotor 1204 to rotate.

Referring to Figs. 13A-13B, the pump assembly 500 can include a bearing base 1302. The bearing base 1302 is a common base between the motor 502 and the pump 504. The bearing base 1302 includes part of the motor 502 and part of the pump 504. The printed circuit board 1202 that controls the motor 502 can be mounted on the bearing base 1302. The bearing base 1302 has a first recess 1306, or a first hollow body 1306, extending from a motor-facing surface 1308 and a second recess 1310, or a second hollow body 1310, extending from a pump-facing surface 1312. The shaft 1208 is received within the first hollow body 1306. The outer rotor 1318 is received within the second hollow body 1310. The motor stator 1304 is mounted around an outer surface of the first hollow body 1306. The motor stator 1304 is fixed. The motor rotor 1204 is positioned around the motor stator 1304. The shaft 1208 is coupled to the motor rotor 1204. The shaft 1208 extends through the motor stator 1304, the first hollow body 1306, and the bearing base 1302 to couple with the pump 504 within the second hollow body 1310. The common bearing base 1302 can improve the alignment of the shaft 1208 extending between the motor 502 and the pump 504, and reduce the overall size, mass, and complexity of the pump assembly 500 which can increase the lifetime of the pump assembly 500.

In this implementation, the bearing base 1302 is a high hardness material such as ceramic or tungsten carbide. The shaft 1208 is also a high hardness material such as a nickel alloy or silicon carbide.

When bearing base 1302 is metal, the pump 504 can be assembled using processes such as laser welding, soldering, adhesive bonding, or mechanical clamping, to couple metal to metal. When the bearing base 1302 is a ceramic, the pump 504 can be assembled using processes such as brazing, adhesive bonding, or mechanical clamping.

The shaft 1208 is coupled to the rotor hub 1206 by means of a press fit, a weld, or an adhesive. In some implementations, the shaft 1208 and the rotor hub 1206 can be a monolithic piece formed by machining or molding. The shaft 1208 is coupled to a keyed opening 1314 in the pump 504.

The motor 502 includes a printed circuit board 1202 that controls the motor 502. The printed circuit board 1202 is electronically coupled to the PCB 402. The printed circuit board 1202 sends and receives signals such as control signals, command signals, and status signals to the PCB 402. In some implementations, the printed circuit board 1202 includes one or more capacitors and one or more Hall effect sensors. In one embodiment, the printed circuit board 1202 has two capacitors to for positive and negative orientations of the windings of the motor stator 1304. In one embodiment, the printed circuit board 1202 has three Hall effect sensors configured to sense and provide control relative to a magnetic field of the motor 502.

Turning to the pump 504, the pump 504 includes an inner rotor 1316 (shown in Figs. 13A-13B), an outer rotor 1318 (shown in Figs 13A-13B), and a pump cover 1320. The inner rotor 1316 rotates relative to and within the outer rotor 1318 to move fluid between the reservoir body 116 and the inflatable bladders 112a, 112b. In this implementation, the pump 504 is a rotary positive displacement pump. For example, the pump 504 can be a gerotor pump. In other implementations, the pump 504 can be another suitable type of pump. In this implementation, the pump 504 has four lobes; however, in other implementations, the pump 504 can have another suitable number of lobes.

The keyed opening 1314 is centered within the inner rotor 1316. The shaft 1208 mates to the inner rotor 1316. The inner rotor 1316 and the outer rotor 1318 are positioned within the second hollow body 1310. The pump cover 1320 couples to the second hollow body 1310 to seal the inner rotor 1316 and the outer rotor 1318 within the second hollow body 1310. The inner rotor 1316 is rotationally coupled to the shaft 1208, but unconstrained in the axial direction. The magnetic attraction between the motor rotor 1204 and the motor stator 1304 ensures that shaft 1208 remains engaged with the inner rotor 1316 in the axial direction. In some cases, the one or more magnets of the motor rotor 1204 can be offset from center to bias the shaft 1208 toward one end so that, during rotation, it is axially pulled toward the motor stator 1304.

The pump 504 can include a thrust bearing 1322. The thrust bearing 1322 receives the shaft 1208. The thrust bearing 1322 is positioned on an inner surface of the pump cover 1320. The thrust bearing 1322 allows the shaft 1208 to rotate relative to the pump cover 1320 with reduced friction. The terminating end of the shaft 1208 can have a crown or radius shape that engages the thrust bearing 1322 to further reduce friction. In this implementation, the thrust bearing 1322 is a sapphire jeweled bearing. In other implementations, the thrust bearing 1322 can be a different synthetic jeweled stone or other suitable bearing material.

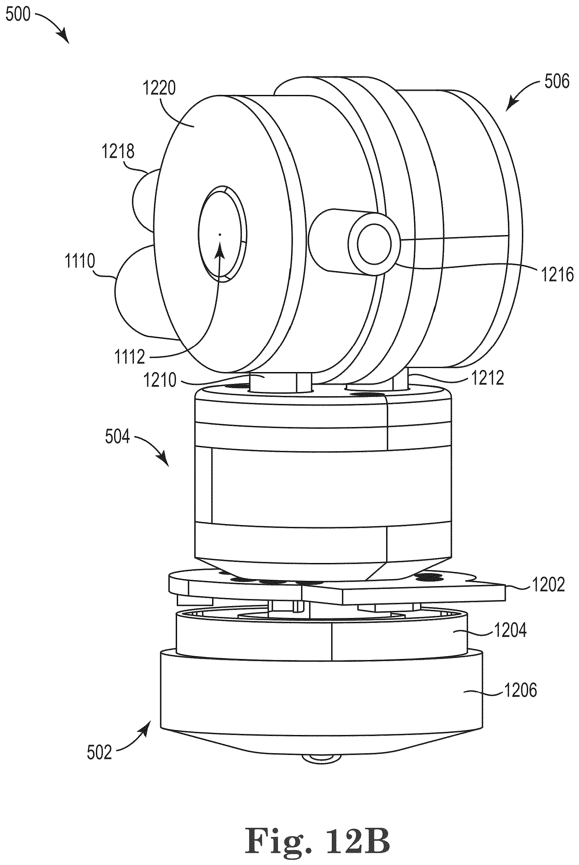

Referring to Figs. 12B and 13A, the pump 504 has a suction conduit 1210 and a discharge conduit 1212. The suction conduit 1210 and the discharge conduit 1212 are coupled to the valve assembly 506. The suction conduit 1210 and the discharge conduit 1212 conduct fluid from the pump 504 to the valve assembly 506. In one implementation shown in Figs. 12A-12B, the suction conduit 1210 and the discharge conduit 1212 extend from end surfaces of the pump 504 to the valve assembly 506. In another implementation, the suction conduit 1210 and the discharge conduit 1212 extend from side surfaces of the pump assembly 504.

A detailed description of a first embodiment of the first valve assembly 1400 follows. The valve assembly 506 shown in Figs. 9, 12A-12B can be provided by the first embodiment of the first valve assembly 1400 (Figs. 14A-14E), which controls the flow of fluid between the reservoir body 116 and the inflatable bladders 112a, 112b. The valve assembly 506 has a valve body 1220 defining an inlet 1214, an outlet 1216, a pressure sensor conduit 1218, and the pressure relief portion 1110. The inlet 1214 is fluidly coupled with the reservoir body 116. The outlet 1216 is fluidly coupled to the inflatable bladders 112a, 112b.

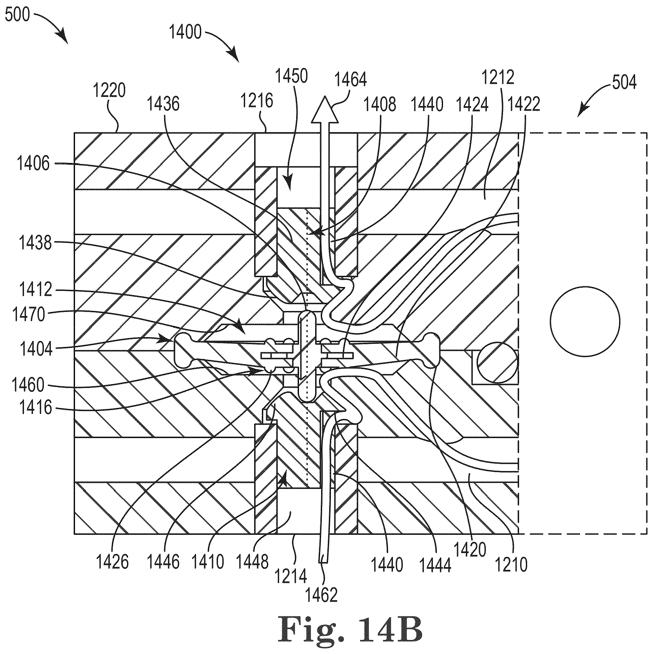

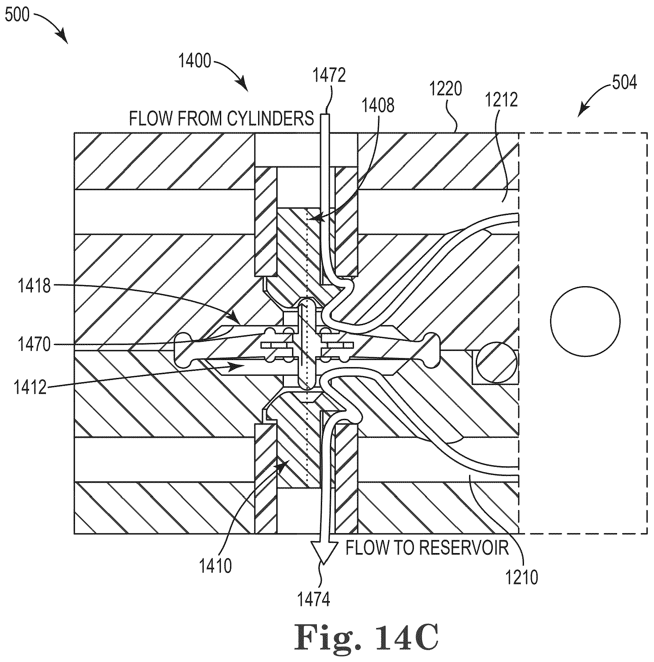

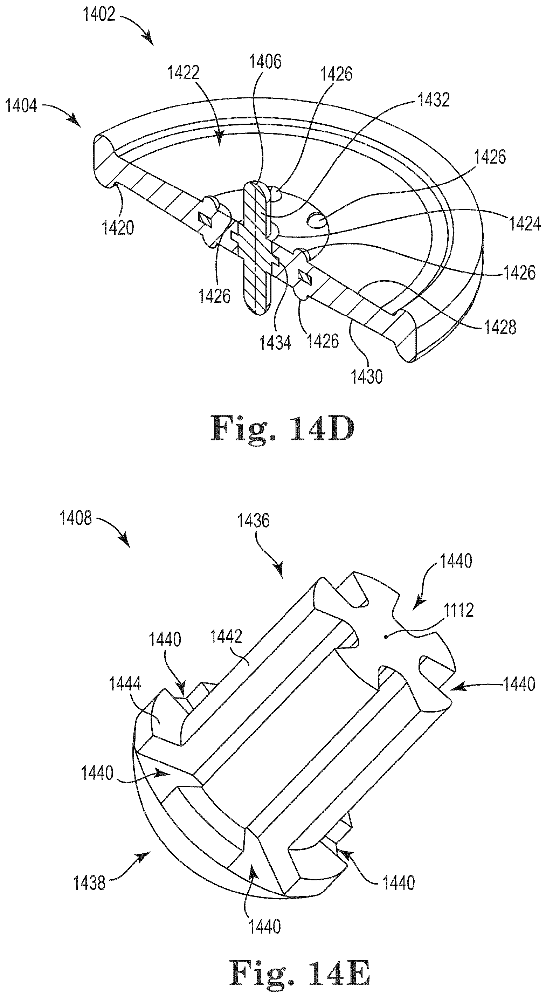

Referring to Figs. 14A-14E, the first valve assembly 1400 includes a double-acting check valve 1402 (best shown in Figs. 14A and 14D). The double-acting check valve 1402 moves responsive to the discharge from the pump 504 to allow flow in one direction and prevent flow in the other, opposite, direction. The double-acting check valve 1402 can move between three positions: a first position 1414 shown in Fig. 14A (undeflected), a second position 1416 shown in Fig. 14B (deflected downward relative to the orientation of Fig. 14B), and a third position 1418 shown in Fig. 14C (deflected upward relative to the orientation of Fig. 14C) to control flow through the first valve assembly 1400 between the reservoir body 116 and the inflatable bladders 112a, 112b.

When the pump 504 is not operating, the double-acting check valve 1402 is initially in the first position 1414 (i.e., a neutral position) preventing flow through the first valve assembly 1400 as shown in Fig. 14A. When the pump 504 is operating in a first direction to flow fluid from the reservoir body 116 to the inflatable bladders 112a, 112b, the double-acting check valve 1402 moves to the second position 1416, allowing the fluid to flow from the pump 504 to the inflatable bladders 112a, 112b as shown in Fig. 14B. When the pump 504 is operating in a second direction opposite the first direction to flow fluid from the inflatable bladders 112a, 112b to the reservoir body 116, the double-acting check valve 1402 moves to the third position 1418, allowing the fluid to flow from the inflatable bladders 112a, 112b through the pump 504 and back to the reservoir body 116, as shown in Fig 14C.

The double-acting check valve 1402 has a membrane 1404, a center pin 1406, a first poppet 1408, and a second poppet 1410 positioned within the valve body 1220. The membrane 1404, the center pin 1406, the first poppet 1408, and the second poppet 1410 are shown in cross-section in Figs. 14A-14C, and in perspective views in Figs. 14D and 14E. The membrane 1404 and the center pin 1406 are positioned in a cavity 1412 within the valve body 1220. The membrane 1404 and the center pin 1406 move within the cavity 1412 responsive to a pressure differential across the membrane 1404 from discharge of the pump 504 to move the poppets 1408, 1410, as described in more detail in reference to Figs. 14A-4C.

The membrane 1404 is a flexible disk constructed from an elastic material. In this implementation, the membrane 1404 is a silicone or a thin metallic film. In other implementations, the membrane 1404 can be a different suitable material that flexes in response to a pressure differential from discharge of the pump 504.

As best shown in Fig. 14D, the membrane 1404 includes a ridge 1420, an interior portion 1422, a center void 1424 to accommodate the center pin 1406, and nubs 1426 projecting away in both directions from the membrane 1404. The ridge 1420 is positioned on an outer perimeter of the membrane 1404. The ridge 1420 is fixed within the valve body 1220. The interior portion 1422 extends from the ridge 1420 to the center void 1424. The interior portion 1422 has a first surface 1428 and a second surface 1430. The center void 1424 is defined through the interior portion 1422 and extends from the first surface 1428 to the second surface 1430. Some of the nubs 1426 are positioned on the first surface 1428 about the center void 1424 and some of the nubs 1426 are positioned on the second surface 1430 about the center void 1424. The nubs 1426 space a portion of the interior portion 1422 of the respective first surface 1428 or second surface 1430 from the cavity 1412 when the membrane 1404 is in either the second position 1416 or the third position 1418, allowing flow past the interior portion 1422 between the pump 504 and the associated first poppet 1408 or second poppet 1410. The membrane 1404 can be over-molded to couple to the center pin 1406.

The membrane 1404 can deflect in the cavity 1412 in either direction based on the pressure differential generated by the pump 504. The first surface 1428 can be in fluid communication with the inflatable bladders 112a, 112b based on the position of the first poppet 1408. The second surface 1430 can be in fluid communication with the reservoir body 116 based on the position of the second poppet 1410. Since the membrane 1404 deflection is induced by the differential pressure across the pump 504, no external venting of one side of the pump 504 or membrane 1404 is required to move the membrane 1404. Thus, the differential pressure across the membrane 1404 is not limited by available atmospheric pressure since the pump assembly 500 is in the fluid volume of the reservoir body 116. The differential pressure across the membrane 1404 corresponds to the pressure output of the pump 504. For a given membrane 1404 size, this allows greater deflection forces to act against the poppets 1408, 1410. The membrane 1404 is symmetrical and therefore operates bidirectionally with the flow of the pump 504 but prevents flow when the pump 504 is off.

The force generated by the membrane center pin 1406 is approximately equal to the net pressure on the membrane 1404 multiplied by the surface area of the membrane 1404, minus the force required to bend the membrane 1404 within the cavity 1412. The membrane 1404 can be actuated with increased efficiency when a bending force is decreased. The force to move the respective first or second poppet 1408, 1410 that the center pin 1406 must overcome to move the first poppet 1408 is equal to the net pressure from the inflatable bladders 112a, 112b and negative side of the pump 504 multiplied by the projected contact area between the poppet 1408 and valve seat 1446a. Increasing the area of the membrane 1404 greater than the contact area can increase reliability. In this implementation, the area of the membrane 1404 is at least 1.5 times larger than the contact area between the poppet 1408 and the valve seat 1446a.

The center pin 1406 extends from the first surface 1428 and the second surface 1430 of the membrane 1404 to contact one or both of the first poppet 1408 or the second poppet 1410 based on the position of the membrane 1404. As the membrane 1404 moves, the center pin 1406 and the pressure differential generated by the pump 504 operates to alter the positions of both the first poppet 1408 and the second poppet 1410 as described in more detail in reference to Figs. 14A-14C. The center pin 1406 is coupled to the membrane 1404 and has a cylindrical body 1432 and a disc 1434. The disc 1434 extends from the cylindrical body 1432 to couple with or integrate with the interior portion 1422 of the membrane 1404. The disc 1434 bisects the cylindrical body 1432. The disc 1434 couples the center pin 1406 to the membrane 1404 within the center void 1424 of the membrane 1404. The ends of the center pin 1406 are rounded to engage the associated first poppet 1408 or the second poppet 1410.

The center pin 1406 can include a portion of the bypass pathway 1112 (Fig. 11). For example, the center pin 1406 can have conduit extending through the cylindrical body 1432 to allow bypass fluid to flow through the double-acting check valve 1402.

The poppets 1408, 1410 move responsive to the force from the center pin 1406 and the pressure differential generated by the pump 504. The poppets 1408, 1410 control fluid flow through the valve body 1220 between the reservoir body 116 and the inflatable bladders 112a, 112b.

Referring to Figs. 14A-14C and 14E, the poppets 1408, 1410 have a body 1436 and a head 1438. The body 1436 can also be referred to as a stem. The head 1438 extends from the body 1436. The body 1436 orients the poppets 1408, 1410 within the conduits of the valve body 1220 (Fig. 12B). The poppets 1408, 1410 have channels 1440 extending longitudinally along an outer surface 1442 of the body 1436 and axially along a rear surface 1444 of the head 1438. The channels 1440 allow fluid to flow between the poppets 1408, 1410 and the valve body 1220. The head 1438 is a hemispherical shape to seal against a valve seat 1446a, b of the valve body 1220. The valve seats 1446a, b are generally conical to receive the head 1438.

The poppets 1408, 1410 can be a monolithic rigid or a semi-rigid material such as a hard plastic or a corrosion resistant metal. In some implementations, the poppets 1408, 1410 can be a composite of different materials for the body 1436 and the head 1438.

In suitable alternative implementations, the first valve assembly 1400 can include one or more biasing springs positioned within the valve body 1220 and in contact with the poppets 1408, 1410. The biasing springs can push the respective first poppet 1408 or second poppet 1410 toward the associated valve seat 1446a or 1446b, respectively, to maintain the associated first poppet 1408 or the second poppet 1410 in contact with the associated valve seat 1446a, b until the force applied by the center pin 1406 overcomes the spring force.

The poppets 1408, 1410 can include a portion of the bypass pathway 1112 (Fig. 11). For example, the poppets 1408, 1410 can have conduit extending through the body to allow bypass fluid to flow through valve body 1220.

Referring to Figs. 14A-14C, the valve body 1220 defines a valve discharge conduit 1450 fluidically coupled to the outlet 1216 and the pump discharge conduit 1212. The first poppet 1408 is positioned between the valve discharge conduit 1450 and the pump discharge conduit 1212. The valve body 1220 defines a valve suction conduit 1448 fluidically coupled to the inlet 1214 and the pump suction conduit 1210. The second poppet 1410 is positioned between the valve suction conduit 1448 and the pump suction conduit 1210.

Referring to Fig. 14A and Fig. 14E, the pump 504 is off. The fluid pressure in the inflatable bladders 112a, 112b pushes against the body 1436 of the first poppet 1408 holding the head 1438 of the first poppet 1408 against the first valve seat 1446a, preventing fluid from flowing from the inflatable bladders 112a, 112b to the pump 504 as shown by arrow 1452. Likewise, the fluid pressure in the reservoir body 116 pushes against the body 1436 of the second poppet 1410 holding the head 1438 of the second poppet 1410 against the second valve seat 1446b, preventing fluid from flowing from the reservoir body 116 to the pump 504 as shown by arrow 1454. The membrane 1404 is in the first position 1414, generally centered within the cavity 1412 with the ends of the pin 1406 spaced apart from the respective heads 1438 of the first and second poppets 1408, 1410. Flow through the first valve assembly 1400 is prevented, as illustrated by the notional "X" on the first and second poppets 1408, 1410. Thus, when the inflatable bladders 112a, 112b are inflated and the pump 504 is off, fluid contained within the inflatable bladders 112a, 112b cannot escape from the inflatable bladders 112a, 112b and pressure within the inflatable bladders 112a, 112b is maintained for use for the desired period of time, even with normal pressure from use on the inflatable bladders 112a, 112. Some fluid may pass through the bypass pathway 1112; the pressure sensor 312 and control from the PCB 402 (Fig. 9) senses the pressure decrease due to the loss of fluid through the bypass pathway 1112 and operates the motor 502 and the pump 504 to maintain a desired set pressure in the inflatable bladders 112a, 112b. When the inflatable bladders 112a, 112b are deflated and the pump 504 is off, fluid cannot be forced out of the reservoir body 116 through the first valve assembly 1400 due to abdominal pressure or gravity which could cause an inadvertent inflation of the inflatable bladders 112a, 112b.

Referring to Fig. 14B, the pump 504 is on and inflating the inflatable bladder 112a, 112b with fluid from the reservoir body 116. The pump 504 is operating in a first, forward direction. The pump 504 is rotating in a first direction, for example, clockwise. The pump 504 is producing a positive pressure in the discharge conduit 1212 and a negative pressure in the suction conduit 1210. The fluid flows through the pump 504 in the direction of arrow 1456. The suction pressure in the suction conduit 1210 pulls the double-acting check valve 1402 in the downward direction until the nubs 1426 on the membrane 1404 contact the surface 1460 of the cavity 1412, so the membrane 1404 is in the second position 1416. The nubs 1426 on the second surface 1430 maintain the second surface 1430 spaced apart from the surface 1460 of the cavity 1412 to provide a flow channel for the fluid. The cylindrical body 1432 of the center pin 1406 contacts the head 1438 of the second poppet 1410 and imparts a force on the second poppet 1410, moving the second poppet 1410 in the downward direction. The nubs 1426 on the second surface 1430 contacting the surface 1460 and the center pin 1406 moving the second poppet 1410 opens a suction flow path 1462 from the reservoir body 116 to the pump 504. The pump 504 generates a positive pressure in the pump discharge conduit 1212, drawing fluid into the pump discharge conduit 1212. The positive pressure and fluid flow likewise forces the membrane 1404 to the second position 1416, or the downward direction. The fluid in the discharge conduit 1212 also draws the first poppet 1408 opposite direction, or the upward direction, opening a discharge flow path 1464 from the pump 504 to the inflatable bladders 112a, 112b.

When the pump 504 is turned off, the pressure across the membrane 1404 approximately equalizes and the differential pressure approaches zero, and a result, the membrane 1404 moves from the second position 1416 to the undeflected first position 1414. The higher pressure in the inflatable bladders 112a, 112b moves the first poppet 1408 to contact the first valve seat 1446a. The fluid pressure in the reservoir body 116 moves the second poppet 1410 to the second valve seat 1446b. Flow through the first valve assembly 1400 is blocked as shown in Fig. 14A.

Referring to Fig. 14C, the pump 504 is on and deflating the inflatable bladders 112a, 112b by moving fluid from the inflatable bladders 112a, 112b to the reservoir body 116. The pump 504 is operating in a second direction opposite the first direction, i.e., a reverse direction. The pump 504 is rotating in the second direction, for example, counterclockwise. The pump 504 is producing a negative pressure in the discharge conduit 1212 and a positive pressure in the suction conduit 1210. The fluid flows through the pump 504 in the direction of arrow 1466. The suction pressure in the discharge conduit 1212 pulls the double-acting check valve 1402 in the upward direction until the nubs 1426 on the membrane 1404 contact the surface 1470 of the cavity 1412, moving the membrane 1404 from the first position 1414 to the third position 1418. The nubs 1426 maintain the first surface 1428 spaced apart from the surface 1470 of the cavity 1412 to allow the fluid to pass. The center pin 1406 contacts the head 1438 of the first poppet 1408 and imparts a force on the first poppet 1408, moving the first poppet 1408 in the upward direction. The nubs 1426 in contact with the surface 1470 and the center pin 1406 moving the first poppet 1408 opens a suction flow path 1472 from the inflatable bladders 112a, 112b to the pump 504. The pump 504 generates a negative pressure in the pump discharge conduit 1212, flowing fluid into the pump discharge conduit 1212 from the inflatable bladders 112a, 112b. The negative pressure pulls the membrane 1404 to the third position 1418 (the upward direction). The pump 504 discharges the fluid into the pump suction conduit 1210. The fluid in the pump suction conduit 1210 on the second surface 1430 aids the suction forces on the first surface 1428 to force the membrane 1404 to the third position 1418. The positive fluid pressure in the pump suction conduit 1210 also moves the second poppet 1410 in the opposite, or downward direction, opening a discharge flow path 1474 from the pump 504 to the reservoir body 116.

When the pump 504 is turned off, the pressure across the membrane 1404 approximately equalizes and the differential pressure approaches zero, and a result, the membrane 1404 moves from the third position 1418 to the first position 1414. The higher pressure in the reservoir body 116 moves the second poppet 1410 to contact the second valve seat 1446b. The fluid pressure in the inflatable bladders 112a, 112b moves the first poppet 1408 to the first valve seat 1446a. Flow through the first valve assembly 1400 is secured as shown in Fig. 14A.

The bypass pathway 1112 can include the check valve 1116 (Fig. 11). The check valve 1116 can be positioned at a suitable location along the bypass pathway 1112. The check valve 1116 allows flow in a single direction. In this implementation, the check valve 1116 only allows flow from the inflatable bladders 112a, 112b to the reservoir body 116. Including the check valve 1116 in the bypass pathway 1112 can allow the inflatable bladders 112a, 112b to slowly deflate, while a higher pressure in the reservoir body 116 is maintained without fluid flowing from the reservoir body 116 to the inflatable bladders 112a, 112b through the bypass pathway 1112.

In this implementation, the check valve 1116 is an umbrella check valve or a duckbill check valve. In other implementations, the check valve 1116 can be a suitable type of check valve.

The first valve assembly 1400 includes the pressure relief portion 1110 (Fig. 11), which provides mechanical overpressure protection at the discharge of the pump 504. The pressure relief portion 1110 includes a ported plug 1222 (Fig. 12A), a spring, and a ball. The ported plug 1222, the spring, and the ball are positioned within the body of the first valve assembly 1400. The spring holds the ball in contact with the body of the first valve assembly 1400 until the discharge pressure of the pump 504 exceeds a discharge pressure threshold. In the implementation of Fig. 11, the discharge pressure threshold is twenty-five pounds per square inch (psi-g). In other implementations, another suitable discharge pressure threshold may be used.

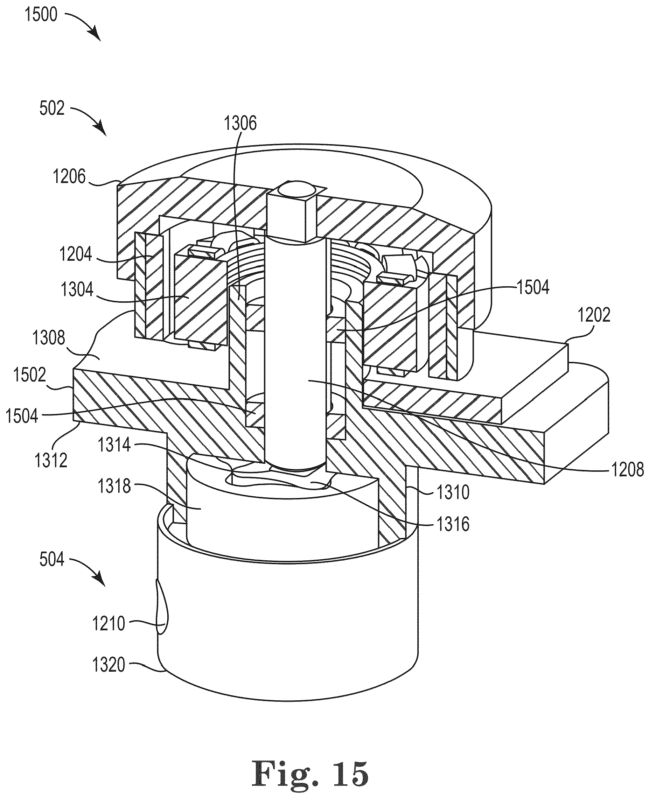

Fig. 15 is another pump assembly 1500, generally similar to the pump assembly 500. The pump assembly 1500 includes the motor 502, the pump 504, and a bearing base 1502. The bearing base 1502 is made from ceramic or metal such as titanium or steel. The pump assembly 1500 includes bearings 1504 inserted into the bearing base 1502. The bearings 1504 provide a mating or bearing surface for the shaft 1208. The bearings 1504 can be roller bearings, jewel bearings such as sapphire or synthetic jewels, or another suitable type of bearing. The pump cover 1320 can be coupled to the bearing base 1502 by laser welding, soldering, adhesive bonding, mechanical clamping, or another suitable means of coupling.

Referring to Figs. 9, 12A-12B, and 16A-16F, the second valve assembly 1600 (a second embodiment of the valve assembly 506) controls the flow of fluid between the reservoir body 116 and the inflatable bladders 112a, 112b. The second valve assembly 1600 is coupled to the pump 504 and mounted to the seal 300 as shown in Fig. 9. The second valve assembly 1600 is in fluid contact with the fluid in the reservoir body 116.

Referring to Figs. 16A-16F, the second valve assembly 1600 has a valve body 1602 having a suction housing 1604, a discharge housing 1606, a membrane 1608 positioned between the suction housing 1604 and the discharge housing 1606, a first poppet 1610, and second poppet 1612. The first poppet 1610 is positioned between the membrane 1608 and the suction housing 1604. The second poppet 1612 is positioned between the membrane 1608 and the discharge housing 1606.

The valve body 1602, the membrane 1608, the first poppet 1610, and the second poppet 1612 form a double acting check valve to control fluid flow between the reservoir body 116 and the inflatable bladders 112a, 112b. The "suction" in suction housing 1604 and the "discharge" in discharge housing 1606 refer to the respective direction of fluid flow to and from the pump 504 when moving the fluid between the reservoir body 116 and the inflatable bladders 112a, 112b. The pump 504 is configured to operate in two directions, one direction for filling the inflatable bladders 112a, 112b and an opposite direction for deflating the inflatable bladders 112a, 112b.

The valve body 1602 includes a suction housing lid 1618 and a discharge housing lid 1620. The suction housing lid 1618 is coupled to the suction housing 1604 opposite the membrane 1608. The discharge housing lid 1620 is coupled to the suction housing 1604 opposite the membrane 1608. The suction housing lid 1618 defines the inlet 1214. The discharge housing lid 1620 defines a void 1622.

The second valve assembly 1600 includes a cap 1624. The cap 1624 is placed within the void 1622 and is coupled to the discharge housing lid 1620. The cap 1624 includes the orifice 1114.

The second valve assembly 1600 includes a screen 1626, which can be useful in filtering or removing particulate matter in the fluid. The screen 1626 is positioned between the cap 1624 and the discharge housing 1606. In some cases, the screen 1626 is a 10 µm reverse Dutch screen.

The suction housing 1604 has an outer hollow cylinder 1628 and an inner structure 1630 positioned within the outer hollow cylinder 1628. The outer hollow cylinder 1628 and the inner structure 1630 direct fluid flow through the suction housing 1604. The inner structure 1630 includes an inner hollow cylinder 1632, a poppet valve seat 1634 positioned within the inner hollow cylinder 1632, and a wall 1636 extending between the inner hollow cylinder 1632 and the outer hollow cylinder 1628. The wall 1636 defines one or more voids 1644 extending therethrough. In this implementation, the outer hollow cylinder 1628 and the inner hollow cylinder 1632 are generally concentric. However, in other implementations, the outer hollow cylinder 1628 and the inner hollow cylinder 1632 can have any suitable size, shape, or orientation.

The suction housing 1604 has a first suction chamber 1638, a second suction chamber 1640, and a third suction chamber 1642. The first suction chamber 1638 is defined within the inner hollow cylinder 1632 by the suction housing lid 1618, the inner hollow cylinder 1632, and the poppet valve seat 1634. The inlet 1214 is fluidly coupled to the inner hollow cylinder 1632. A first portion 1646 of the first poppet 1610 is positioned within the inner hollow cylinder 1632 and movable relative to the poppet valve seat 1634 within the inner hollow cylinder 1632 to control fluid through the first suction chamber 1638.

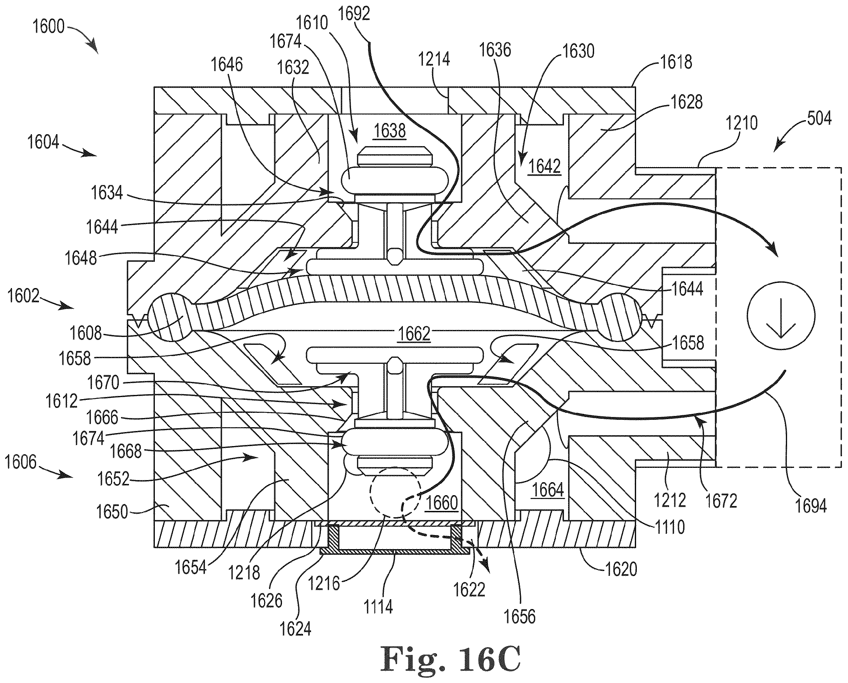

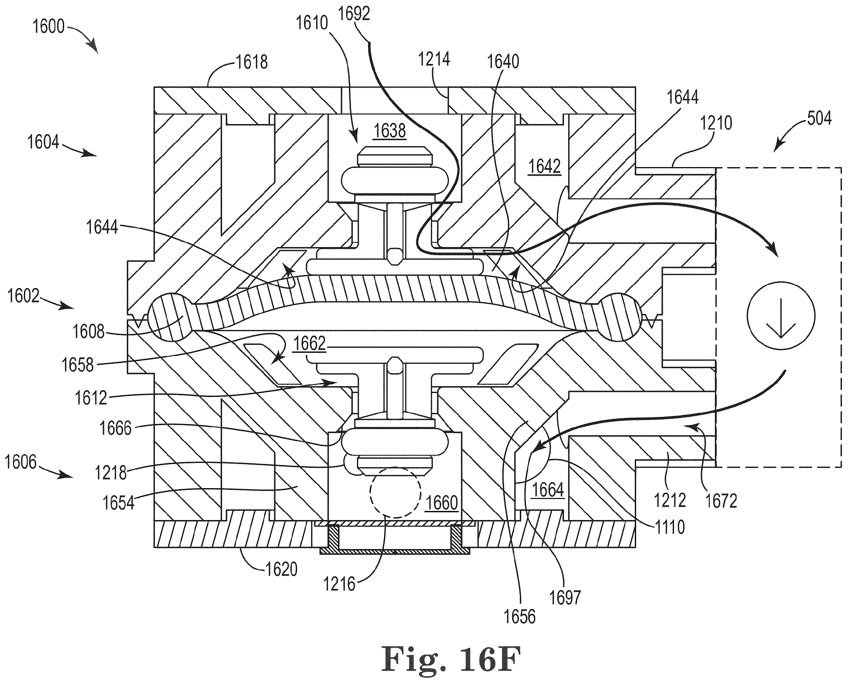

The second suction chamber 1640 is defined by the wall 1636 and the membrane 1608. The first poppet 1610 extends through the valve seat 1634 into the second suction chamber 1640. A second portion 1648 of the first poppet 1610 is positioned within the second suction chamber 1640. The membrane 1608 moves in and out of contact with the first poppet 1610 based on a pressure differential between the first suction chamber 1638 and the second suction chamber 1640 as described in more detail in reference to Figs. 16C-16D and 16F.

The third suction chamber 1642 is defined between the outer hollow cylinder 1628, the inner structure 1630, the wall 1636, and the suction housing lid 1618. The third suction chamber 1642 is fluidly coupled to the second suction chamber 1640 by the one or more voids 1644 in the wall 1636. The suction conduit 1210 extends through the outer hollow cylinder 1628 and is fluidly coupled to the third suction chamber 1642.

The discharge housing 1606 has an outer hollow cylinder 1650 and an inner structure 1652 positioned within the outer hollow cylinder 1650. The outer hollow cylinder 1650 and the inner structure 1652 to direct fluid flow through the discharge housing 1606. The inner structure 1652 includes an inner hollow cylinder 1654, a poppet valve seat 1666 positioned within the inner hollow cylinder 1654, and a wall 1656 extending between the inner hollow cylinder 1654 and the outer hollow cylinder 1650. The wall 1656 defines one or more voids 1658 extending therethrough. In this implementation, the outer hollow cylinder 1650 and the inner hollow cylinder 1654 are generally concentric. However, in other implementations, the outer hollow cylinder 1650 and the inner hollow cylinder 1654 can have any suitable size, shape, or orientation.

The discharge housing 1606 has a first discharge chamber 1660, a second discharge chamber 1662, and a third discharge chamber 1664. The first discharge chamber 1660 is defined within the inner hollow cylinder 1654 by the discharge housing lid 1620, the inner hollow cylinder 1654, and the poppet valve seat 1666. The orifice 1114 is fluidly coupled to the inner hollow cylinder 1654. A first portion 1668 of the second poppet 1612 is positioned within the inner hollow cylinder 1654 and movable relative to the poppet valve seat 1666 within the inner hollow cylinder 1654 to control fluid through the first discharge chamber 1660. The pressure sensor conduit 1218 extends from the first discharge chamber 1660 to the outer hollow cylinder 1650. The volume within the pressure sensor conduit 1218 is fluidly isolated from the second discharge chamber 1662 since the pressure sensor conduit 1218 passes from the first discharge chamber 1660 through the second discharge chamber 1662 to the outlet of the pressure sensor conduit 1218. The pressure sensor conduit 1218 provides a sensing pathway through the valve body 1602 to the pressure sensor 312 (Fig. 9) on the opposite side of the seal 300. The outlet conduit 1672 extends from the first discharge chamber 1660 to the outlet 1216 on the outer hollow cylinder 1650 to provide a path for fluid to move from the pump 504 to the first and second inflatable bladders 112a, 112b. The volume within the outlet conduit 1672 is fluidly isolated from the second discharge chamber 1662 as the outlet conduit 1672 passes from the first discharge chamber 1660 through the second discharge chamber 1662 to the outlet 1216.

The second discharge chamber 1662 is defined by the wall 1656 and the membrane 1608. The second poppet 1612 extends through the valve seat 1666 into the second discharge chamber 1662. A second portion 1670 of the second poppet 1612 is positioned within the second discharge chamber 1662. The membrane 1608 moves in and out of contact with the second poppet 1612 based on a pressure differential between the first discharge chamber 1660 and the second discharge chamber 1662 as described in more detail in reference to Figs. 16C-16D and 16F.

The third discharge chamber 1664 is defined between the outer hollow cylinder 1650, the inner structure 1652, the wall 1656, and the discharge housing lid 1620. The third discharge chamber 1664 is fluidly coupled to the second discharge chamber 1662 by the one or more voids 1658 in the wall 1656. The discharge conduit 1212 extends from the outer hollow cylinder 1650 and is fluidly coupled to the third discharge chamber 1664. The pressure relief portion 1110 extends from the outer hollow cylinder 1650 and is fluidly coupled to the third discharge chamber 1664.

The membrane 1608 is positioned between the suction housing 1604 and the discharge housing 1606. The membrane 1608 is captured in grooves in the suction housing 1604 and the discharge housing 1606. The membrane 1608 is a flexible pressure barrier between the second suction chamber 1640 and the second discharge chamber 1662. The membrane 1608 flexes responsive to a pressure differential between the second suction chamber 1640 and the second discharge chamber 1662.

As the membrane 1608 flexes toward the suction housing 1604, the membrane 1608 contacts the first poppet 1610. The membrane 1608 contacts the second portion 1648 of the first poppet 1610 and repositions the first poppet 1610 from a normally closed position to an open position. The first portion 1646 of the first poppet 1610 is moved off the first valve seat 1634, fluidly coupling the first suction chamber 1638 and the second suction chamber 1640.

As the membrane 1608 flexes toward the discharge housing 1606, the membrane 1608 contacts the second poppet 1612. The membrane 1608 contacts the second portion 1670 of the second poppet 1612 and repositions the second poppet 1612 from a normally closed position to an open position. The first portion 1668 of the second poppet 1612 is moved off the poppet valve seat 1666, fluidly coupling the first discharge chamber 1660 and the second discharge chamber 1662.

The membrane 1608 has an outer ridge surrounding a generally disc shaped interior portion. The membrane 1608 is constructed from an elastic material. In this implementation, the membrane 1608 is a silicone or a thin metallic film. In other implementations, the membrane 1608 can be a different suitable material.

Referring to Figs. 16A-16F, the second valve assembly 1600 can include an o-ring 1674. The o-ring 1674 can be coupled to the first portions 1646, 1668 of the first poppet 1610 and the second poppet 1612 to improve sealing of the first poppet 1610 and the second poppet 1612 on the respective valve seats 1634, 1666.

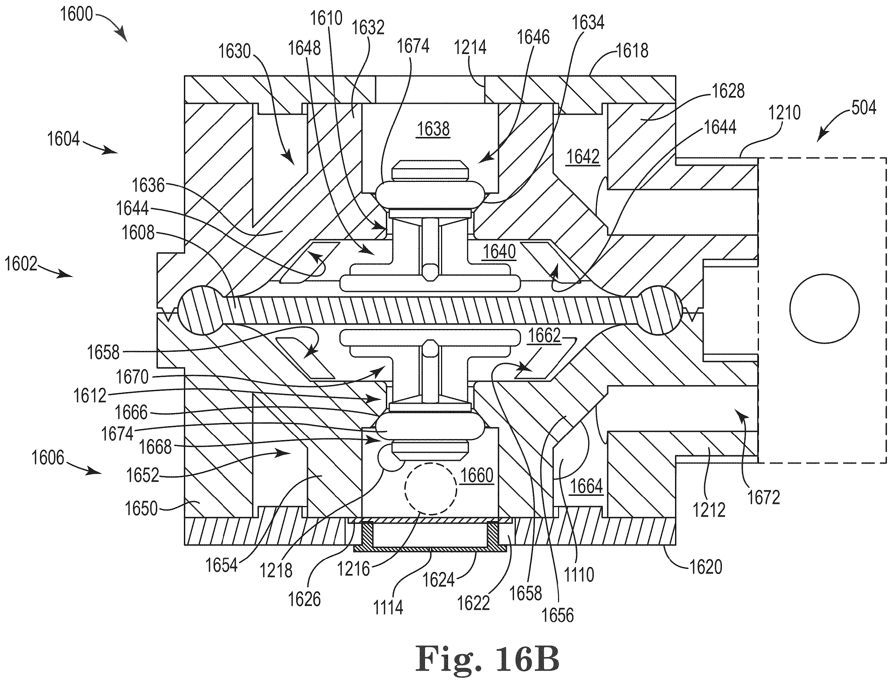

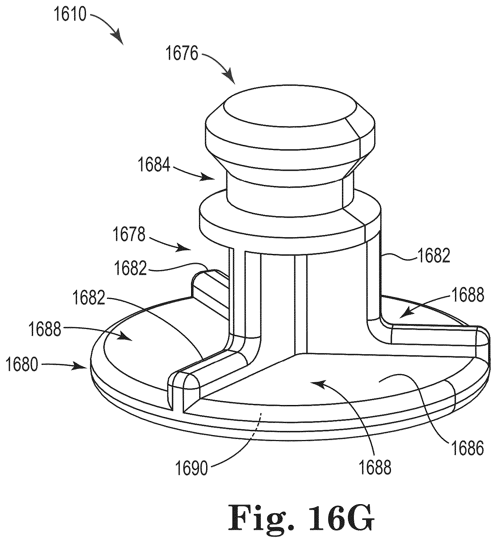

Referring to Fig. 16G and Fig. 16B, the first poppet 1610 and the second poppet 1612 share a similar structure, and the description of the first poppet 1610 informs the structure of the second poppet 1612. The first poppet 1610 has a head 1676, a shaft 1678, a disc 1680, and ribs 1682. The head 1676 has an o-ring groove 1684 sized to accept the o-ring 1674. When the o-ring 1674 is not employed, the head 1676 and the o-ring groove 1684 are optionally sealed against the valve seat 1634 based on the position of the first poppet 1610 relative to the poppet valve seat 1634. The shaft 1678 extends from the head 1676 to the disc 1680. When the first poppet 1610 is positioned in the suction housing 1604, the shaft 1678 extends through the valve seat 1634 between the first suction chamber 1638 and the second suction chamber 1640. When the first poppet 1610 is positioned in the suction housing 1604, the disc 1680 is positioned within the second suction chamber 1640. The ribs 1682 extend along the shaft 1678 and a head-facing surface 1686 of the disc 1680. The ribs 1682 align the first poppet 1610 relative to the inner structure 1652 and provide channels 1688 or valleys 1688 for the fluid to flow between the first poppet 1610 and the inner structure 1652. The disc 1680 has membrane-facing surface 1690. The membrane-facing surface 1690 is opposite the head-facing surface 1686. The membrane-facing surface 1690 receives the membrane 1608 when the membrane 1608 has been repositioned. The second poppet 1612 is substantially similar to the first poppet 1610.

Referring to Figs. 16B-16F, the second valve assembly 1600 controls fluid flow between the pump 504, the reservoir body 116, and the inflatable bladders 112a, 112b. Flow through the second valve assembly 1600 is based on whether or not the pump 504 is rotating, the direction of rotation of the pump 504, and the pressure at the discharge of the pump 504 (i.e., in the third discharge chamber 1664).

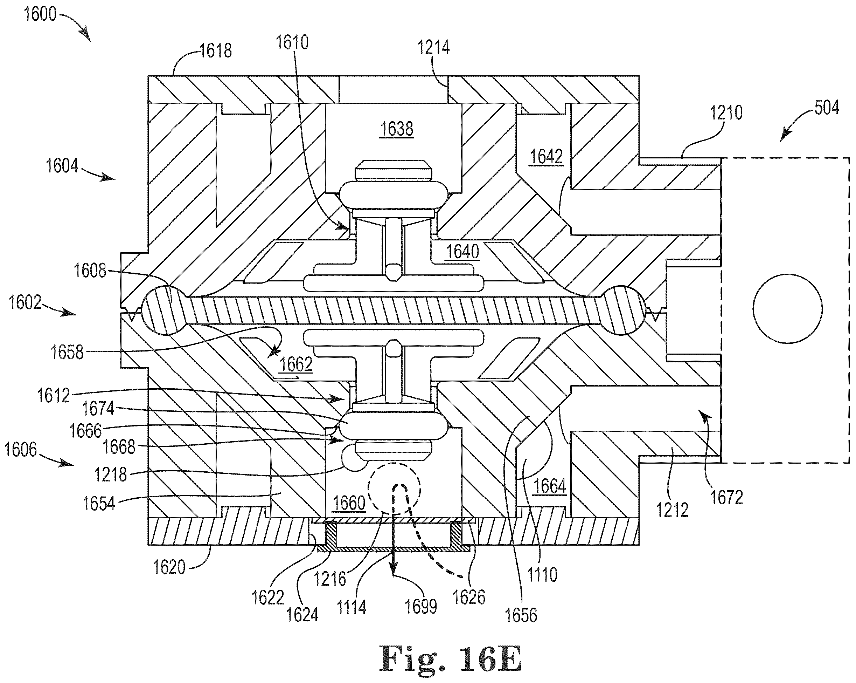

Referring to Fig. 16B and Fig. 16G, the pump 504 is not operating, or the differential pressure across the membrane 1608 is less than a differential pressure threshold. The membrane 1608 is in a normal resting position and has not flexed or has not flexed enough to contact either the first poppet 1610 or the second poppet 1612. The fluid and fluid pressure in the internal cavity 250 (Fig. 8) of the reservoir body 116 passes through the inlet 1214 into the first suction chamber 1638 and presses the head 1676 of the first poppet 1610, holding the head 1676 on the poppet valve seat 1634 and preventing fluid flow between the first suction chamber 1638 and the second suction chamber 1640. The fluid and fluid pressure in the inflatable bladders 112a, 112b passes through the main tubing 212 and the outlet 1216 and is on the head 1676 of the second poppet 1612, holding the head 1676 of the second poppet 1612 on the poppet valve seat 1666 and preventing fluid flow between the first discharge chamber 1660 and the second discharge chamber 1662. Some fluid in the inflatable bladders 112a, 112b can pass into the first discharge chamber 1660 and out the orifice 1114 back to the reservoir body 116 as described in more detail in reference to Fig. 16E. In some implementations, a biasing spring may be used to keep poppets 1610 and 1612 normally closed against their respective valve seats

Referring to Figs. 16C and 16G, the pump 504 is operating to inflate the inflatable bladders 112a, 112b. The pump 504 flows fluid from the reservoir body 116 to the inflatable bladders 112a, 112b. Operation of the pump 504 in the first direction produces a suction in the suction housing 1604, displacing the first poppet 1610 off the seat 1634 to allow flow through the suction housing 1604. Specifically, the pump 504 draws a suction in the third suction chamber 1642, causing a pressure decrease in the third suction chamber 1642 and the second suction chamber 1640 through the one or more voids 1644. The suction pressure in the second suction chamber 1640 moves the membrane 1608 toward the suction housing 1604 and into engagement with the membrane-facing surface 1690 on the disc 1680 of the first poppet 1610. The head 1676 of the first poppet 1610 moves off the first valve seat 1634. Moving the first poppet 1610 off the first valve seat 1634 opens a first inflation flow path 1692. The first inflation flow path 1692 extends from the internal cavity 250 of the reservoir body 116 through the inlet 1214 into the first suction chamber 1638, past the poppet valve seat 1634 into the second suction chamber 1640, from the second suction chamber 1640 through the one or more voids 1644 to the third suction chamber 1642, and from the third suction chamber 1642 through the suction conduit 1210 to the pump 504. At the same time, the operation of the pump 504 in the first direction produces a discharge in the discharge housing 1606, displacing or repositioning the second poppet 1612 to allow flow through the discharge housing 1606. Specifically, the pump 504 discharges into the third discharge chamber 1664, causing a pressure increase in the third discharge chamber 1664 and the second discharge chamber 1662 through the one or more voids 1658. The discharge pressure in the second discharge chamber 1662 moves the membrane 1608 away from the discharge housing 1606 (e.g., upward) and forces the head 1676 of the second poppet 1612 away from the second valve seat 1666. The head 1676 of the second poppet 1612 moves off the poppet valve seat 1666. Moving the second poppet 1612 off the poppet valve seat 1666 opens a second inflation flow path 1694. The second inflation flow path 1694 extends from the pump 504, through the outlet conduit 1672 to the third discharge chamber 1664, from the third discharge chamber 1664 to the second discharge chamber 1662 through the one or more voids 1658, from the second discharge chamber 1662 past the poppet valve seat 1666 though the valleys 1688 in the second poppet 1612, past the head 1676 of the second poppet 1612 into the first discharge chamber 1660, out the first discharge chamber 1660 through the outlet 1216 to the inflatable bladders 112a, 112b, thereby inflating the inflatable bladders 112a, 112b.

**In** an alternative embodiment, the second poppet 1612 is absent from discharge housing 1606 such that flow path 1694 is always open in both directions. This may be preferable in situations where fluid in inflatable bladders 112a and 112b is held back by another valve elsewhere along the fluid path between valve assembly 1600 and inflatable bladders 112a, 112b. Utilizing this alternative embodiment of the valve assembly without the second poppet allows a scrotal deflate button to be added to allow manual deflation. The scrotal deflate button is familiar to users of penile implants and implementing a scrotal deflate button (implanted in the scrotum) allows the user to beneficially choose to manually deflate the inflatable bladders.

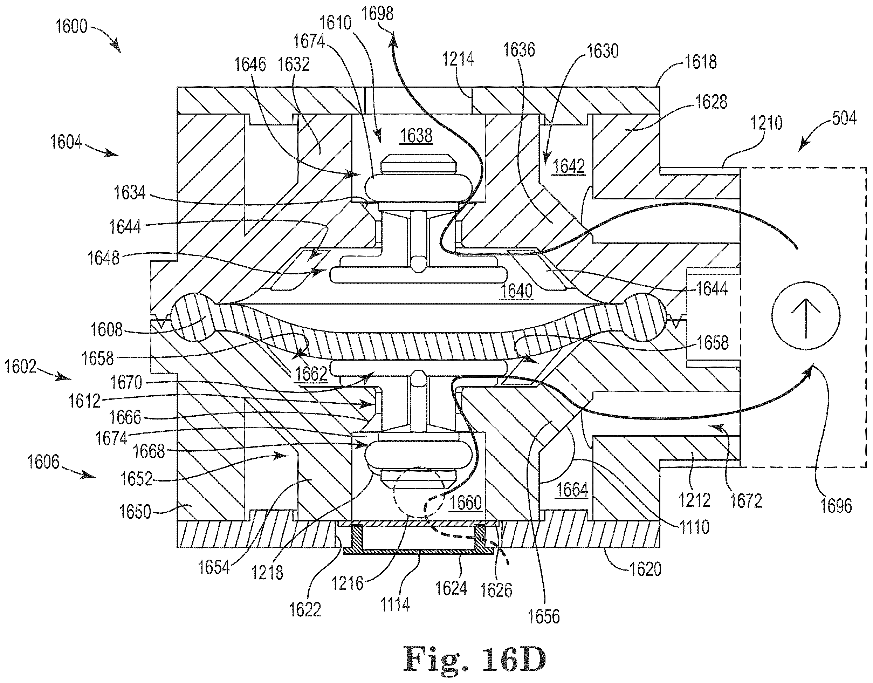

Referring to Figs. 16D and 16G, the pump 504 is operating to deflate the inflatable bladders 112a, 112b. The pump 504 flows fluid from the inflatable bladders 112a, 112b to the reservoir body 116. The pump 504 is operated in the second direction, opposite the first direction. Operation of the pump 504 in the reverse direction reverses fluid direction through the pump 504. The pressure differential across the pump 504 between the discharge conduit 1212 and the suction conduit 1210 causes the membrane 1608 to deflect from the resting position toward the discharge housing 1606 and opening flow through the second valve assembly 1600. Fluid enters the second valve assembly 1600 through the outlet 1216 from the inflatable bladders 112a, 112b and exits the second valve assembly 1600 through the inlet 1214 to the reservoir body 116.

Specifically, the pump 504 produces a suction in the discharge housing 1606, displacing or repositioning the second poppet 1612 to allow flow through the discharge housing 1606 by drawing a suction in the third discharge chamber 1664, causing a pressure decrease in the third discharge chamber 1664 and the second discharge chamber 1662 through the one or more voids 1658. The suction pressure in the second discharge chamber 1662 moves the membrane 1608 toward the discharge housing 1606 and into engagement with the membrane-facing surface 1690 on the disc 1680 of the second poppet 1612. The head 1676 of the second poppet 1612 moves off the poppet valve seat 1666. Moving the second poppet 1612 off the poppet valve seat 1666 opens a first deflation flow path 1696. The first deflation flow path 1696 extends from the inflatable bladders 112a, 112b through the outlet 1216 into the first discharge chamber 1660, and past the poppet valve seat 1666 and the second poppet 1612 into the second discharge chamber 1662. The first deflation flow path 1696 continues from the second discharge chamber 1662 through the one or more voids 1658 into the third discharge chamber 1664 to the outlet conduit 1672 and into the pump 504.

At the same time, the operation of the pump 504 in the second direction produces a discharge in the suction housing 1604, displacing or repositioning the first poppet 1610 to allow flow through the suction housing 1604. The pump 504 discharges into the third suction chamber 1642, causing a pressure increase in the third suction chamber 1642 and the second suction chamber 1640 through the one or more voids 1644. The discharge pressure in the second suction chamber 1640 moves the membrane 1608 away from the suction housing 1604 and forces the head 1676 of the first poppet 1610 off the poppet valve seat 1634. Moving the first poppet 1610 off the poppet valve seat 1634 opens a second deflation flow path 1698. The second deflation flow path 1698 extends from the pump 504, through the suction conduit 1210 to the third suction chamber 1642, from the third suction chamber 1642 to the second suction chamber 1640 through the one or more voids 1644, from the second suction chamber 1640 past the poppet valve seat 1634 though the valleys 1688 in the first poppet 1610, past the head 1676 of the first poppet 1610 into the first suction chamber 1638, and out the first suction chamber 1638 through the inlet 1214 to reservoir body 116, thereby deflating the inflatable bladders 112a, 112b.

Referring to Fig 16E, the eIPP 110 is in a pressure hold mode. The pump 504 is not operating, and the differential pressure across the membrane 1608 is less than the differential pressure threshold. The inflatable bladders 112a, 112b are pressurized, and fluid can slowly leak back from the inflatable bladders 112a, 112b to the reservoir body 116. The fluid flows into the second valve assembly 1600 through the outlet 1216 into the first discharge chamber 1660, then through the orifice 1114 in the cap 1624 to the reservoir body 116 as shown by pressure hold flow path 1699. The second poppet 1612 is on the poppet valve seat 1666, sealing the first discharge chamber 1660 from the second discharge chamber 1662. Since pressure in the inflatable bladders 112a, 112b is monitored using the pressure sensor 428, the controller of the PCB 402 activates the motor 502 on an as-needed basis to maintain a set pressure, causing the pump 504 to further inflate the inflatable bladders 112a, 112b periodically to raise pressure in the inflatable bladders 112a, 112b back to the setpoint level as described in reference to Fig. 16C.

Referring to Fig. 16F, the second valve assembly 1600 is operating in a safety relief mode. If pressure in the third discharge chamber 1664, measured as the discharge pressure of the pump 504, exceeds a safety pressure threshold, the pressure relief portion 1110 (i.e., a safety relief valve such as a spring-loaded check valve) diverts fluid back to the reservoir body 116 along safety-relief flow path 1697.

The eIPP 110 is compact and suitable to be implanted through a single incision. A single incision is one and only one opening in the skin that is usually retracted open to provide access into the penis and abdomen and other parts of the body. One implantation procedure for the eIPP 110 is described below and includes implantation of the inflatable penile implant 112 in the penis, implantation of the reservoir assembly 114 posterior the transversalis fascia, and implantation of the antenna assembly 120 anterior the transversalis fascia along a beltline of the patient, for example, along an area of the pelvis between the pubic bone and an elevation of the ilium in a location that allows discretely recharging the battery or power source of the eIPP 110.

One procedure begins by placing the patient on a surgical table in a dorsal recumbent position. The surgeon might elect to extend the surgical table to form a plateau or flat surface along the mons pubis of the patient. The groin is shaved and prepped from the umbilicus to mid-thigh with a suitable anti-bacterial surgical solution such as 3M DuraPrep^{™} Surgical Solution, available from Solventum, St. Paul, MN or HIBICLENS Antiseptic Skin Cleanser, available from Molnlycke Health Care, Norcross, GA. The surgical team will determine the appropriate anesthetic, and at times selects a pudendal nerve block applied to the lateral corpora and injected into the Alcock's canal. It can be helpful to cause an artificial erection of the penis by injecting approximately 60 cc of liquid, which can be saline or a solution of saline and lidocaine, for full hydrodilatation of each corpus cavernosum of the penis. The hydrodilatation of the penis allows for identification of the penile anatomy prior to incision.

The compactness of the eIPP 110 allows for convenient implantation with an infrapubic approach, although a penoscrotal approach is acceptable, as determined by the surgeon. Regarding the infrapubic procedure, an infrapubic incision is formed laterally about one finger width above or superior a top surface of the penis into the skin of the pubis, which is above and away from the penis. The infrapubic incision is preferably sized only large (or wide) enough to accommodate insertion of the reservoir assembly 114. A retractor is inserted into the incision to expose the corpora. The tunica is retracted with stay sutures. A scalpel blade is used to incise and form bilateral corporotomies wide enough to accommodate a width of the selected inflatable bladder 112, and preferably not wider. The surgeon will measure a depth of the penis distal and proximal, for example, with a Furlow introducer or other suitable measurement aid, which guides a selection of a length of the inflatable bladders 112a, 112b. Each corpus cavernosum is suitably dilated to a size that will accept the measured and selected inflatable body 112a, 112b. Each inflatable body 112a, 112b is implanted into the dilated corpus cavernosum with the tubing housing 238 approximately at a level of the corporotomies. A Keith needle may be used to direct the inflatable bladders 112a, 112b through the associated corpus cavernosum, where the Keith needle is carried by a string that is coupled to the inflatable body 112a, 112b. The needle pierces the glans, and the string is used to pull the inflatable body 112a, 112b through and along an axis of the corpus cavernosum. The stay sutures may be used to close the corporotomy and are placed distal the tubing 200, 202, as determined by the surgeon.

A speculum is inserted through the infrapubic incision to bluntly dissect tissue and create a pocket for placement of the reservoir assembly behind the transversalis fascia. A finger can be inserted through the incision to identify the transversalis fascia anteriorly with an open space posteriorly. The reservoir assembly 114 is placed into the open space posterior the transversalis fascia and cephalad the infrapubic incision. It can be useful to place the reservoir assembly 114 as far cephalad as possible away from the pelvic girdle to create a separation between placement of the reservoir assembly 114 and the antenna assembly 120, which avoids the potential of the reservoir assembly 114 undesirably electrically interfering with the antenna assembly 120 during charging or recharging of the battery. Preferably, the reservoir assembly 114 is implanted in a space dissected away from the tissue plane, located posterior to the muscle, and sutured in place to surrounding tissue.

The antenna assembly 120 is implanted below the navel of the umbilical region by tunneling an instrument in a subdermal path to form an open space anterior the transversalis fascia. The antenna assembly 120 is inserted into the open space anterior the pubic bone. In one embodiment, the antenna assembly 120 is inserted into a space anterior the pubic bone and behind the transversalis fascia. In a preferred embodiment, the antenna assembly 120 is inserted subdermally into a space above the muscle and fat. The antenna assembly 120 is used for battery charging, and it is preferred that the patient can discreetly recharge the eIPP 110. To this end, it can be desirable to place the antenna assembly 120 along a beltline of a user of the eIPP 110.

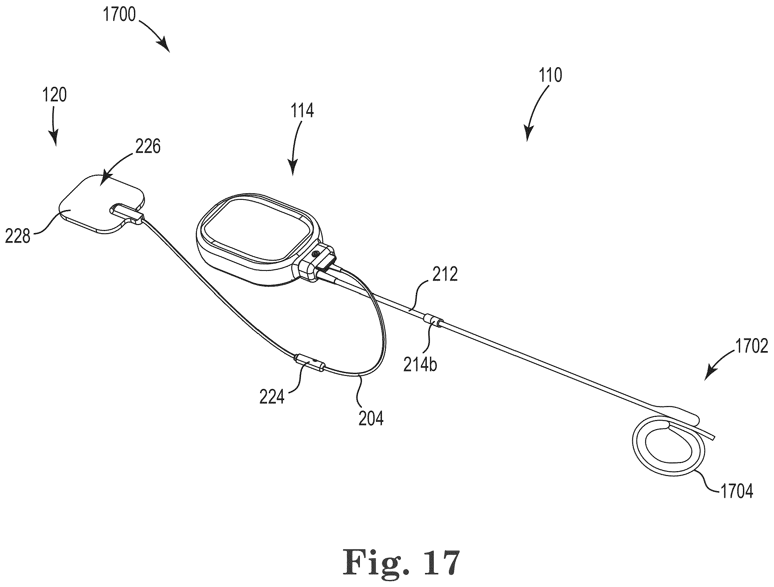

Fig. 17 is a perspective view of one embodiment of an electronic prosthesis provided as an electronic artificial sphincter 1700. The electronic artificial sphincter 1700 includes the reservoir assembly 114 described above with the tubing 212 connected to an implantable prothesis, which in the case of an artificial sphincter is usefully provided as an inflatable cuff 1704. The electronic artificial sphincter 1700 is suitable for implantation in the body with the cuff 1704 surrounding the urethra of the patient, in which case the electronic artificial sphincter 1700 is an electronic artificial urinary sphincter (eAUS) 1700. The electronic artificial sphincter 1700 is suitable for implantation in the body with the cuff 1704 surrounding the anal sphincter area of the patient, in which case the electronic artificial sphincter 1700 is an electronic artificial fecal incontinence sphincter 1700. In these situations, the cuff 1704 can be filled with liquid to inflate the cuff 1704 to close off a body lumen or tube, whether the urethra or the anus. The reservoir assembly 114 can be electronically operated by the user to move liquid out of the cuff 1704 and into the reservoir body 116 (Fig. 8) to allow the body lumen to open and pass body waste. The reservoir assembly 114 operates to move liquid from the reservoir body 116 back into the cuff 1704 to provide the user with a dry, continent state.

The eAUS 1700 includes the main tubing 212 and the antenna assembly 120 attachable to the reservoir assembly 114, as described above, and an artificial urinary sphincter (AUS) 1702 provided as a cuff 1704 that is attachable to the main tubing 212. The AUS 1702 is shown with the cuff 1704 wrapped and secured in a circular form as it would be after surgical implantation around a urethra. The connector 214b allows the surgeon to adjust a length of the main tubing 212 to a length measured intraoperatively, where the measured length extends a distance from a location of where the reservoir assembly 114 is implanted to a location where the cuff 1704 is implanted, usually near the prostatic urethra or the bulbar urethra, depending upon surgeon preference.

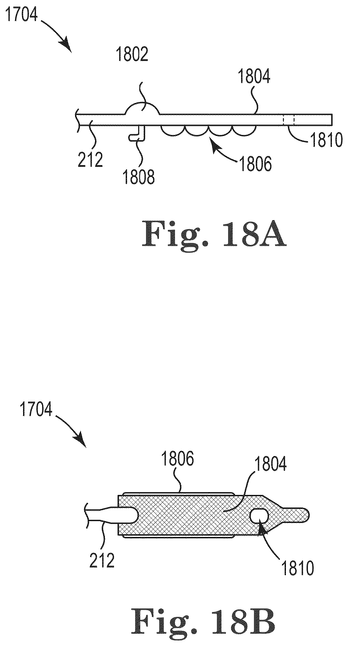

Fig. 18A is a side view and Fig. 18B is a top view of the cuff 1704 in an open configuration prior to surgical implantation. The cuff 1704 has a manifold 1802 attached to a backing 1804. The manifold 1802 communicates with an inflatable pillow 1806; a latch 1808 is configured to securely couple with an opening 1810 in the backing 1804; and the tubing 212 is connected to the manifold 1802. During use, after implantation, the cuff 1704 surrounds the urethra and the inflatable pillow 1806 is pressurized to a steady state pressure that expands the inflatable pillow 1806, which occludes or closes the urethra. In one implementation, the patient uses the patient interface 104 (the fob 104a or the handheld activating device 104b of Fig. 1) to active the motor 502 and operate the pump 504 to move liquid within the eAUS 1700. For example, the inner rotor 1316 rotates relative to and within the outer rotor 1318 to move an amount of liquid out of the inflatable pillow 1806 of the cuff 1704 and into the reservoir body 116, which decreases the pressure in the cuff 1704 (and increases the liquid pressure in the reservoir body 116), which removes the occlusive pressure of the cuff around the urethra, which opens the urethra to allow the patient to pass urine through the urethra.

The steady state pressure useful to provide an occluding pressure in the cuff 1704 is in a range from 50 - 80 cm H2O (38 - 59 mm Hg). One common pressure range suitable for most patients is between 45 - 51 mm Hg. Some patients can be relieved of incontinence with a lower pressure range between 38 - 44 mm Hg. Some patients have a higher urine leak pressure and are beneficially treated with a higher-pressure range between 52 - 59 mm Hg.

The cuff 1704 can be made of an elastomer. The cuff 1704 can have lengths in a range from 3.5 cm to 11.0 cm, for example in 0.5 cm increments. The cuff 1704 can have a width in a range from 1.5 to 4 cm, with one useful width for the cuff being 2.0 cm. In other embodiments, the cuff 1704 can have other suitable dimensions.

The eAUS 1700 can include any one or more of the features and operations of the electronic implantable prosthesis described in reference to FIGS. 1-16G. The eAUS 1700 can operate in a closed configuration with a steady state pressure condition preventing flow through the urethra or an open configuration allowing flow through the urethra.

In the steady state pressure condition, no flow moves through the valve assembly 506. The pressure through the eAUS 1700 is maintained at the steady state pressure in the range 38 - 59 mm Hg to close the cuff 1704 around the urethra and prevent urine leakage for the user.

When the user desires to pass urine, a signal is sent by the user to the controller on the PCB 402 to operate the pump 504 in a direction to move liquid from the cuff 1704 back to the reservoir body 116, allowing the fluid to flow through the pump 504 back to the reservoir body 116. The pressure in the cuff 1704 can then be reduced, allowing the cuff 1704 to relax or open, which allows urine to flow through the urethra. The additional liquid retained in the reservoir body 116 creates a pressure in the reservoir body 116 that is greater than the steady state pressure.

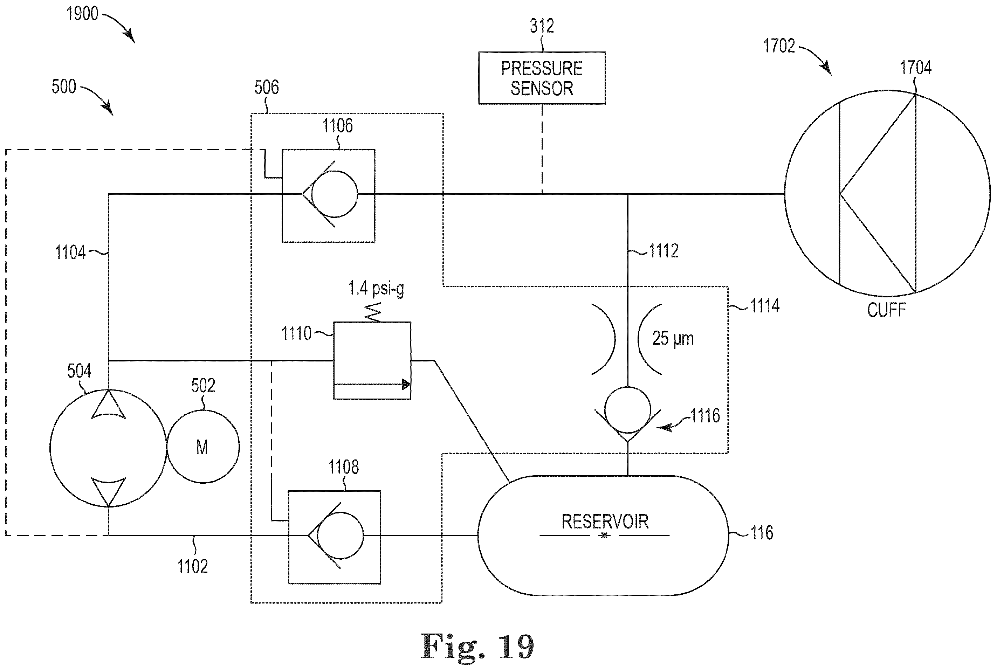

Fig. 19 is one embodiment of a piping and instrumentation diagram 1900 that functionally illustrates the components of the eAUS 1700 including the reservoir body 116, the cuff, and the pump assembly 500 (the motor 502, pump 504, and the valve assembly 506). The valve assembly 506 of the eAUS 1700 includes a similar bypass pathway 1112, except the check valve 1116 is rotated by 180 degrees relative to the check valve 1116 of Fig. 11 to allow the displaced liquid to move from the reservoir body 116 back into the cuff 1704 with no input from the user. For example, after passing urine, the cuff 1704 may suitably close relatively slowly as the bladder is empty, so no active refill of the cuff 1704 is called for, which is one way to ensure long battery life. The bypass pathway 1112 allows the additional liquid retained in the reservoir body 116 to leak back into the cuff 1704. The liquid moved from the cuff to the reservoir body 116 is at a higher pressure inside the reservoir body 116 relative to the cuff 1704. The bypass pathway allows the liquid in the reservoir body 116 to move or seep or leak out of the reservoir body 116 to the cuff 1704 to again pressurize the cuff 1704 at the steady state pressure, which results in the cuff 1704 occluding the urethra and providing the user with a continent state. The bypass pathway 1112 is continually open allowing a portion of the fluid that was moved out of the cuff 1704 to flow back to the reservoir body 116 with no patient input.

In an alternate implementation of pressurizing the cuff 1704 to provide the user with a continent (closed urethra) state, the pump assembly 500 (the motor 502, pump 504, and the valve assembly 506) is activated by the user to actively move liquid from the reservoir body 116, through the pump 504, and back to the cuff 1704. During active refilling of the inflatable pillow 1806 of the cuff 1704, the pump 504 is operating in a first direction, or forward direction. The pump 504 is rotating in a first direction, for example, clockwise. The pump 504 is producing a positive pressure in the discharge conduit 1212 and a negative pressure in the suction conduit 1210. The fluid flows through the pump 504 from the reservoir body 116, opening the valve assembly 506. The pump 504 generates a positive pressure in the pump discharge conduit 1212, flowing fluid into the pump discharge conduit 1212, and flowing fluid from the pump 504 to the cuff 1704.

Although specific embodiments have been illustrated and described herein, it will be appreciated by those of ordinary skill in the art that a variety of alternate and/or equivalent implementations may be substituted for the specific embodiments shown and described without departing from the scope of the present invention. This application is intended to cover any adaptations or variations of medical devices as discussed herein.

## Claims

1. A body implantable reservoir assembly adapted to move liquid relative to an implantable prosthesis, the reservoir assembly comprising:
a reservoir body adapted to contain the liquid; and
an electronic assembly disposed in the reservoir body and adapted to move the liquid between the reservoir body and the implantable prosthesis, the electronic assembly comprising:
a printed circuit board and a battery hermetically sealed inside a canister, and
a pump assembly comprising a motor electrically coupled with the printed circuit board and the battery, a pump coupled with the motor, and a valve assembly
coupled with the pump;
wherein, when the reservoir body contains the liquid, the motor, the pump, and the valve assembly are immersed in the liquid.

2. The reservoir assembly of claim 1, wherein the motor comprises a rotor and a stator and the rotor and the stator are surrounded by the liquid.

3. The reservoir assembly of claim 1, wherein the printed circuit board and the battery are hermetically sealed inside the canister by a seal secured to the canister, and the seal comprises an electrical feedthrough that forms an electrical connection between the motor and the printed circuit board and the battery.

4. The reservoir assembly of claim 1, wherein the electronic assembly further comprises:
an end cap enclosing the reservoir body, where the end cap comprises a tube adapted to fluidically couple an inflatable bladder of the implantable prosthesis with the reservoir body.

5. The reservoir assembly of claim 1, wherein the electronic assembly further comprises:
an end cap enclosing the reservoir body, where the end cap comprises a tube adapted to fluidically couple an inflatable cuff of the implantable prosthesis with the reservoir body.

6. The reservoir assembly of claim 1, wherein the electronic assembly comprises a cover secured to the canister;
wherein the cover comprises a channel adapted to allow the liquid in the reservoir body to enter the cover and surround the motor.

7. The reservoir assembly of claim 1, wherein the motor comprises a shaft coupled with the pump, with the motor and the shaft adapted to rotate in the liquid to obviate sealing the shaft from the liquid.

8. The reservoir assembly of claim 1, wherein the motor comprises a shaft coupled with an inner rotor of the pump, with the inner rotor of the pump inserted into and rotatable relative to an outer rotor of the pump;
wherein rotation of the inner rotor of the pump moves the liquid between the reservoir body and an inflatable bladder of the implantable prosthesis.

9. The reservoir assembly of claim 1, wherein the valve assembly comprises:
a valve body having a suction housing and a discharge housing;
a membrane positioned between the suction housing and the discharge housing;
a first poppet positioned between the membrane and the suction housing; and
a second poppet positioned between the membrane and the discharge housing;
wherein the suction housing is fluidly coupled to the reservoir body and the discharge housing is configured to be fluidly coupled to an inflatable bladder of the implantable prosthesis.

10. The reservoir assembly of claim 1, wherein the electronic assembly comprises a cover secured to the canister, and the cover comprises a channel adapted to allow the liquid in the reservoir body to enter the cover and contact the valve assembly;
wherein the valve assembly comprises a valve body provided with a bypass pathway;
wherein the bypass pathway is configured to continuously allow a portion of the liquid in an inflatable bladder of the implantable prosthesis to flow to the reservoir body.

11. The reservoir assembly of claim 1, wherein the valve assembly comprises a bypass pathway formed in a body of the valve assembly, with the bypass pathway fluidly communicating between a pair of inflatable bladders of the implantable prosthesis and the reservoir body, the pair of inflatable bladders being implantable into a penis, with the bypass pathway configured to continuously allow a portion of the liquid in the pair of inflatable bladders to flow to the reservoir body.

12. The reservoir assembly of claim 1, wherein the pump assembly comprises a pressure sensor adapted to monitor a pressure in the pair of inflatable bladders, with the motor is adapted to run when the pressure sensor senses a pressure in a pair of inflatable bladders of the implantable prosthesis, the pair of inflatable bladders being implantable into a penis, when said pressure is below a set pressure for the pair of inflatable bladders.

13. The reservoir assembly of claim 1, wherein;
the motor is configured to run in a first direction to move the liquid from the reservoir body into a pair of inflatable bladders of the implantable prosthesis, the pair of inflatable bladders being implantable into a penis;
wherein, when the pair of inflatable bladders is manually squeezed, the motor is configured to run in a second direction opposite of the first direction to regeneratively charge the battery.

14. The reservoir assembly of claim 1, wherein;
after implantation, the motor is configured to run in a first direction to move the liquid from the reservoir body, through the pump, through the valve assembly, and into a pair of inflatable bladders of the implantable prosthesis, the pair of inflatable bladders being implantable into a penis;
and wherein, during an implantation procedure, the motor is configured to run in a second direction opposite of the first direction to intraoperatively evacuate air from the pair of inflatable bladders and the reservoir body to provide evacuated inflatable bladders and an evacuated reservoir body.

15. The reservoir assembly of claim 14, wherein, during an implantation procedure, the motor is configured to run in the second direction to intraoperatively draw the liquid into the evacuated inflatable bladders and the evacuated reservoir body.

16. The reservoir assembly of claim 1, wherein the motor comprises a magnet, and when in a presence of a magnetic field of a magnetic resonance imaging machine, the magnet of the motor creates an induced force, and a combined weight of the reservoir assembly is greater than the induced force to allow the body implantable reservoir assembly to be MRI compatible in a magnetic field having a magnetic flux density in a range from 0.5 T to 1.5 T.

17. The reservoir assembly of claim 1, further comprising:
an end cap sealed to the reservoir body to enclose the electronic assembly inside the end cap and the reservoir body; and
a septum formed in one of the reservoir body and the end cap;
wherein the septum is adapted to self-seal after a needle passes through the septum to deliver an initial amount of liquid to lubricate the pump upon start-up.

18. The reservoir assembly of claim 1, further comprising:
an end cap sealed to the reservoir body to enclose the electronic assembly inside the end cap and the reservoir body; and
an implantable antenna coupled to the end cap and electrically connected to the battery.

19. A system, comprising:
the reservoir assembly of any one of claims 1-18, and
an implantable prosthesis.

20. The system of claim 19, wherein the implantable prosthesis comprises a penile implant comprising an inflatable bladder.

21. The system of claim 20, wherein the penile implant comprises a pair of inflatable bladders.

22. The system of claim 19, when dependent on claim 18, wherein the implantable prosthesis comprises an electronic inflatable penile prosthesis (eIPP), comprising:
a main tubing having a first end coupled to the end cap and a second end formed as a junction splitting the main tubing into pair of tubes; and
a pair of inflatable bladders implantable into a penis, with each one of the pair of inflatable bladders coupled with one of the pair of tubes;
wherein the motor operates in a first direction to fill the pair of inflatable bladders with the liquid and a second direction to drain the liquid from the pair of inflatable bladders into the reservoir body.

23. The system of claim 22, wherein the motor comprises a permanent magnet and a combined weight of the reservoir assembly including the motor and the liquid and the battery and the circuit board configures the eIPP to be MRI compatible in a magnetic field having a magnetic flux density in a range from 0.5 T to 1.5 T.

24. The system of claim 19, wherein the implantable prosthesis comprises an artificial urinary sphincter comprising an inflatable cuff.
